# EUROPEAN PATENT SPECIFICATION

(11) **EP 3 529 380 B1**
(45) Date of publication and mention of the grant of the patent: **29.06.2022**
(21) Application number: 17797209.8
(22) Date of filing: 19.10.2017
(51) Int. Cl.: C12Q 1/6806

(54) **METHODS FOR CHEMICAL LIGATION OF NUCLEIC ACIDS**
VERFAHREN ZUR CHEMISCHEN LIGATION VON NUKLEINSÄUREN
MÉTHODES DE LIGATURE CHIMIQUE D'ACIDES NUCLÉIQUES

(30) Priority: 19.10.2016 US 201662410172 P
(43) Date of publication of application: 28.08.2019
(73) Proprietor: Illumina, Inc., San Diego, CA 92122 (US); Illumina Singapore PTE. Ltd., Singapore 138667 (SG)
(72) Inventor: TEO, Yin Nah, Singapore 138667 (SG); CHEN, Xi-Jun, Forster City CA 94404 (US); KHURANA, Tarun, Forster City CA 94404 (US)
(74) Representative: Murgitroyd & Company
(86) International application number: PCT/US2017/057415
(87) International publication number: WO 2018/075785

(56) References cited:
- WO-A1-2015/177520
- US-A1- 2008 050 731
- US-A1- 2015 051 088
- ROUTH ANDREW ET AL: "ClickSeq: Fragmentation-Free Next-Generation Sequencing via Click Ligation of Adaptors to Stochastically Terminated 3'-Azido c", JOURNAL OF MOLECULAR BIOLOGY, vol. 427, no. 16, 14 August 2015 (2015-08-14), pages 2610-2616, XP029252591, ISSN: 0022-2836, DOI: 10.1016/J.JMB.2015.06.011
- MARIE-LUISE WINZ ET AL: "Site-specific terminal and internal labeling of RNA by poly(A) polymerase tailing and copper-catalyzed or copper-free strain-promoted click chemistry", NUCLEIC ACIDS RESEARCH, vol. 40, no. 10, 16 February 2012 (2012-02-16), pages e78-e78, XP055441625, GB ISSN: 0305-1048, DOI: 10.1093/nar/gks062
- ZHENG YUXUAN ET AL: "Synthesis and evaluation of an alkyne-modified ATP analog for enzymatic incorporation into RNA", BIOORGANIC & MEDICINAL CHEMISTRY LETTERS, PERGAMON, AMSTERDAM, NL, vol. 26, no. 7, 18 February 2016 (2016-02-18), pages 1799-1802, XP029453939, ISSN: 0960-894X, DOI: 10.1016/J.BMCL.2016.02.038
- A. H. EL-SAGHEER ET AL: "Biocompatible artificial DNA linker that is read through by DNA polymerases and is functional in Escherichia coli", PROCEEDINGS OF THE NATIONAL ACADEMY OF SCIENCES, vol. 108, no. 28, 12 July 2011 (2011-07-12), pages 11338-11343, XP055196445, ISSN: 0027-8424, DOI: 10.1073/pnas.1101519108
- El-Sagheer Afaf H. ET AL: "Synthesis and Polymerase Chain Reaction Amplification of DNA Strands Containing an Unnatural Triazole Linkage", Journal of the American Chemical Society, vol. 131, no. 11, 25 March 2009 (2009-03-25), pages 3958-3964, XP055793843, US ISSN: 0002-7863, DOI: 10.1021/ja8065896 Retrieved from the Internet: URL:https://pubs.acs.org/doi/pdf/10.1021/j a8065896>

## Description

### FIELD

This application generally relates to preparing nucleic acid libraries and capturing nucleic acids using chemical ligation.

### BACKGROUND

The low DNA input of single cell applications and cell free DNA applications places a high demand on the conversion efficiency for library preparation for DNA sequencing. The conversion efficiency is largely determined by the enzymatic ligation efficiency of the sequencing adapters to the DNA library of interest. Enzymatic ligation of nucleic acids often has low conversion rate due to low enzymatic ligation efficiency.

A multitude of chemical ligation methods are known, including using various chemicals such as cyanogen bromide. Further, nucleophilic reactions involving phosphorothioates and iodoacetyl, bromoacetyl, tosylate and iodo-modified nucleosides have been described. More recently, click chemistry using 1,3-dipolar cycloaddition based methods have been used in various contexts.

Biocompatible ligation methods are known, including the ligation reaction between 5' iodo and 3' phosphorothioate to form the phosphorothioate linkage and click ligation including reaction between 3' azide and 5' alkyne to form a triazolyl linker. Either a 3' alkynyl and 5' azido- linkage or 3' azido and 5' alkynyl group can be used for click ligation. The former has been shown to have full biocompatibility, as it was faithfully copied by polymerases in a polymerase chain reaction. The latter can also be copied by polymerases but results in 1 skipped base. The base that has the 3' azido modification is not read by the polymerase.

Routh Andrew ET AL: "ClickSeq: Fragmentation-Free Next-Generation Sequencing via Click Ligation of Adaptors to Stochastically Terminated 3'-Azido cDNAs", JOURNAL OF MOLECULAR BIOLOGY, vol. 427, no. 16, 14 August 2015, p. 2610-2616 relates to click ligation of adaptors to 3'-Azido cDNAs.

Currently, sequencing of nucleic acid from small sample sizes and creation of nucleic acid libraries suffers from low efficiency enzymatic ligation efficiency resulting in gaps in the resulting sequencing results. Furthermore, capture of nucleic acids from small samples in and of itself can suffer from low efficiencies associated with enzymatic ligation.

Thus, there is a need in the art for an efficient method to ligate nucleic acids together to increase sequencing output and accuracy. The present invention satisfies this need and provides related advantages as well.

### BRIEF SUMMARY

The present invention is defined in the appended claims. Provided herein, *inter alia,* are methods useful for preparing nucleic acid libraries and capturing nucleic acids from a sample. The methods described herein can improve efficiency in preparing a nucleic acid library from a given sample, including for example, samples with very little amounts of nucleic acid contained in the sample. For example, low conversion rates due to low enzymatic ligation efficiency using standard techniques can be increased using the chemical ligation methods described herein.

Disclosed is a method of preparing a nucleic acid library from a sample comprising:
(i) reacting a sample comprising a plurality of cellular nucleic acids each having a terminal 3'-modified dideoxynucleotide (ddNTP) comprising a 3'-functional moiety capable of participating in a click chemistry reaction with a plurality of adaptor nucleic acids each comprising a terminal 5'-modified ddNTP comprising a compatible 5'-functional moiety capable of participating in a click chemistry reaction with the 3' functional moiety, wherein the 3' functional moiety and 5' functional moiety react to form a modified backbone linkage, thereby forming a plurality of ligated-nucleic acids; and
(ii) amplifying the plurality of ligated-nucleic acids thereby preparing a nucleic acid library from the sample.

Disclosed is a method of preparing a nucleic acid library from a sample comprising:
(i) reacting a sample comprising a plurality of cellular nucleic acids each having a terminal 3'-modified ddNTP comprising a 3'-functional moiety capable of participating in a click chemistry reaction, wherein the 3'-modified ddNTP is incorporated into each of the plurality of cellular nucleic acids by contacting the plurality of cellular nucleic acids with a template independent polymerase, with a plurality of adaptor nucleic acids each comprising a terminal 5'-modified ddNTP comprising a compatible 5'-functional moiety capable of participating in a click chemistry reaction with the 3' functional moiety, wherein the 3' functional moiety and 5' functional moiety react to form a modified backbone linkage, thereby forming a plurality of ligated-nucleic acids; and
(ii) amplifying the plurality of ligated-nucleic acids thereby preparing a nucleic acid library from the sample.

Disclosed is a method of preparing a nucleic acid library from a sample comprising:
(i) reacting a sample comprising a plurality of cellular nucleic acids each comprising a terminal 3'-modified ddNTP comprising a 3'-functional moiety capable of participating in a chemical ligation reaction, wherein the 3'-modified ddNTP is incorporated into each of the plurality of cellular nucleic acids by contacting the plurality of cellular nucleic acids with a template independent polymerase, with a plurality of adaptor nucleic acids each comprising a terminal 5'-modified ddNTP comprising compatible 5'-functional moiety capable of participating in a chemical ligation reaction with the 3' functional moiety, wherein the 3' functional moiety and 5' functional moiety react to form a modified backbone linkage, thereby forming a plurality of ligated-nucleic acids; and
(ii) amplifying the plurality of ligated-nucleic acids thereby preparing a nucleic acid library from the sample.

Disclosed is a method of preparing a nucleic acid library from a sample comprising:
(i) reacting a sample comprising a plurality of cellular nucleic acids each comprising a terminal 3'-modified ddNTP comprising a 3'-functional moiety capable of participating in a click chemistry reaction, wherein the terminal 3'-modified ddNTP is incorporated into each of the plurality of cellular nucleic acids by contacting the plurality of cellular nucleic acids with a template independent polymerase, with a plurality of adaptor nucleic acids each comprising a terminal 5'-modified ddNTP comprising compatible 5'-functional moiety capable of participating in a click chemistry reaction with the 3' functional moiety, wherein the 3' functional moiety and 5' functional moiety react to form a modified backbone linkage, thereby forming a plurality of ligated-nucleic acids;
(ii) contacting the plurality of ligated-nucleic acids to a solid support under conditions for hybridization, wherein the solid support comprises (1) a plurality of capture primers having a nucleic acid sequence complementary to the plurality of adaptor nucleic acids, and (2) a plurality of universal primers;
(iii) extending the plurality of capture primers to produce a plurality of immobilized target nucleic acids complementary to the ligated-nucleic acids;
(iv) annealing the plurality of universal primers to the immobilized target nucleic acids; and
(v) amplifying by polymerase chain reaction (PCR) the plurality of immobilized target nucleic acids.

Disclosed is a method of preparing a nucleic acid library from a sample comprising;
(i) reacting a sample comprising a plurality of cellular nucleic acids each having a terminal 3'-modified ddNTP comprising a 3'-functional moiety capable of participating in a click chemistry reaction, wherein the 3'-modified ddNTP, with a plurality of adaptor nucleic acids attached to a support, each of the plurality of adaptor nucleic acids comprising a terminal 5'-modified ddNTP comprising a compatible 5'-functional moiety capable of participating in a click chemistry reaction with the 3' functional moiety, wherein the 3' functional moiety and 5' functional moiety react to form a modified backbone linkage, thereby forming a plurality of ligated-nucleic acids; and
(ii) amplifying the plurality of ligated-nucleic acids thereby preparing a nucleic acid library from the sample.

Disclosed is a method of preparing a nucleic acid library from a sample comprising;
(i) reacting a sample comprising a plurality of cellular nucleic acids each having a terminal 3'-modified ddNTP comprising a 3'-functional moiety capable of participating in a click chemistry reaction, wherein the 3'-modified ddNTP, with a plurality of molecular tethers, each of the plurality of molecular tethers comprising a functional moiety capable of participating in a click chemistry reaction with the 3' functional moiety, wherein the 3' functional moiety and functional moiety of the molecular tether, thereby forming a plurality of ligated-cellular nucleic acids; and
(ii) amplifying the plurality of ligated-cellular nucleic acids thereby preparing a nucleic acid library from the sample.

Disclosed is also method of preparing a nucleic acid library from a sample, the method comprising:
(i) attaching the plurality of cellular nucleic acids to a solid support under conditions for hybridization, wherein the solid support comprises:
   (A) a plurality of capture primers each having a nucleic acid sequence complementary to the plurality of adaptor nucleic acids; and
   (B) a plurality of 3'-universal primers;
(ii) extending the plurality of capture primers to produce a plurality of immobilized cellular nucleic acids;
(iii) incorporating a terminal 3'-modified ddNTP comprising a 3'-functional moiety capable of participating in a click chemistry reaction into each of the immobilized cellular nucleic acids thereby forming a plurality of 3'-modified cellular nucleic acids;
(iv) reacting the 3'-modified cellular nucleic acids with a plurality of adaptor nucleic acids comprising a 5'-modified ddNTP comprising a compatible 5'-functional moiety capable of participating in a click chemistry reaction with the 3' functional moiety, wherein the 3' functional moiety and 5' functional moiety react to form a modified backbone linkage, thereby forming a plurality of ligated-nucleic acids; and
(v) amplifying the plurality of ligated-nucleic acids thereby preparing a nucleic acid library from the sample.

In yet another aspect disclosed herein is a method of capturing DNA obtained from a limited number of cells for DNA library preparation comprising:
(i) reacting a sample comprising a plurality of cellular DNA fragments comprising a terminal 3'-modified ddNTP comprising a 3'-functional moiety capable of participating in a click chemistry reaction obtained from a single cell, wherein the terminal 3'-modified ddNTP is incorporated into each of the plurality of cellular nucleic acids by contacting the plurality of cellular nucleic acids with a template independent polymerase, with a plurality of adaptor nucleic acids each comprising a terminal 5'-modified ddNTP comprising compatible 5'-functional moiety capable of participating in a click chemistry reaction with the 3' functional moiety, wherein the 3' functional moiety and 5' functional moiety react to form a modified backbone linkage; and
(ii) contacting the plurality of ligated-nucleic acids to a solid support under conditions for hybridization, wherein the solid support comprises (1) a plurality of capture primers having a nucleic acid sequence complementary to the plurality of adaptor nucleic acids, thereby capturing DNA obtained from a single cell.

In another aspect disclosed herein is a method of selectively cleaving a single strand of a double stranded polynucleotide sequence, the method comprising:
(i) preparing a template strand by reacting a first polynucleotide comprising a terminal 3'-modified ddNTP comprising a 3'-functional moiety capable of participating in a click chemistry reaction, wherein the terminal 3'-modified ddNTP is incorporated into the first polynucleotide by contacting the first polynucleotide with a template independent polymerase, with a second polynucleotide comprising a terminal 5'-modified ddNTP comprising compatible 5'-functional moiety capable of participating in a click chemistry reaction with the 3' functional moiety, wherein the 3' functional moiety and 5' functional moiety react to form a modified backbone linkage, wherein the template strand comprises a first restriction site that comprises the modified backbone linkage;
(ii) extending the first or second polynucleotide to produce a double stranded nucleic acid, wherein the complementary strand of the double stranded nucleic acid comprises a second restriction site complementary to the first restriction site;
(iii) contacting the double stranded nucleic acid with a nucleic acid-cleaving enzyme;
(iv) cleaving the double stranded nucleic acid with the nucleic acid-cleaving enzyme, wherein the nucleic acid-cleaving enzyme recognizes the first and second restriction sites and cleaves only at the second restriction site, forming a 5'-primer sequence and a 3'-strand.

### DESCRIPTION OF THE FIGURES

FIG. 1 illustrates TdT incorporation of 3' azido ddNTPs to ssDNA and dsDNA with either blunt ends or an A overhang.
FIG. 2 illustrates TdT incorporation of 3' alkynyl ddNTPs to ssDNA.
FIG. 3 illustrates consecutive TdT and click reaction produced final clicked product (Lane 6). Conditions were as shown in Example described herein.
FIG. 4 illustrates the effect of varying concentrations of TdT enzyme and 3'-azido-dNTP on TdT reaction yield and final click ligation yield.
FIG. 5 illustrates the effect of the length of TdT incubation.
FIG. 6 illustrates consecutive TdT and click ligation with 3'-azido-ddGTP (1.55 µM of template).
FIG. 7 illustrates TdT-assisted, click ligation with 3'-azido-ddGTP (18.8µM).
FIG. 8 illustrates click reaction performed at below freezing temperatures gave a much higher yield compared to reactions performed at room temperature or 70 °C. Reaction conditions: copper(II) sulfate (400 eqv), sodium ascorbate (4000 eqv.), PMDETA (20980 eqv.) and DNA template (0.9 µM).

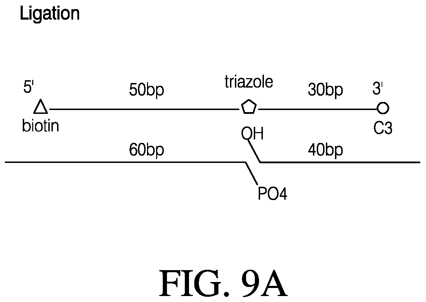

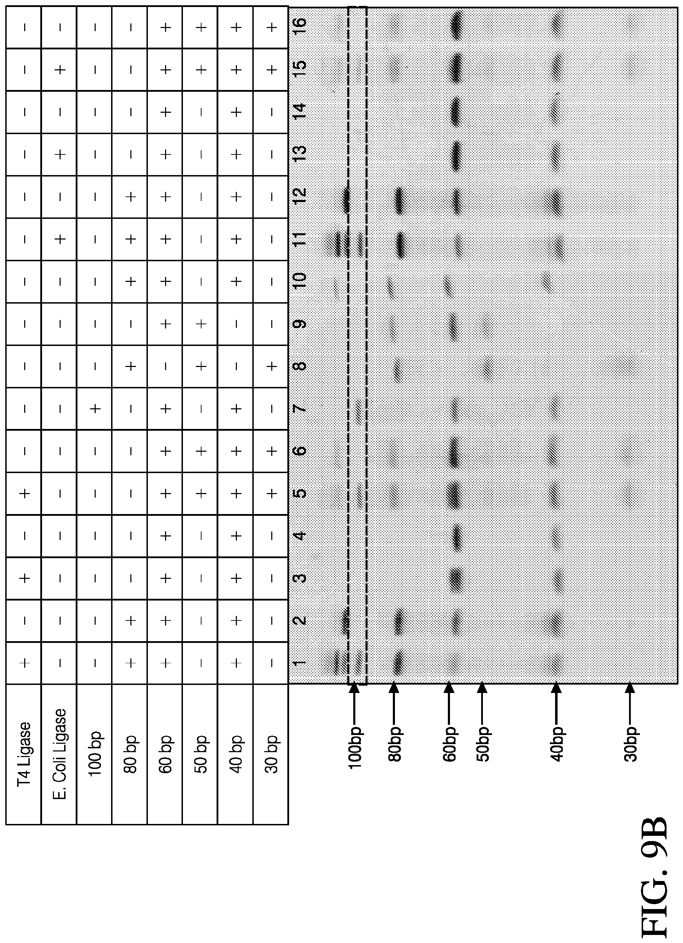

FIG. 9 illustrates a triazole linkage formed upon click ligation is amenable as a template for DNA ligases. FIG. 9A shows the general scheme. FIG. 9B shows the ligation efficiency at various conditions shown demonstrating biocompatibility of the ligation product. (Percent yield using T4 ligase = 89.1%). (Percent yield using E. coli ligase = 49.9%).
FIG. 10 illustrates a click ligation for direct RNA capture and ligation of sequencing adapter.
FIG. 11 illustrates using click ligation for direct library capture.
FIG. 12 illustrates the triazole linkage from click chemistry can be extended by DNA Pol Klenow fragment.

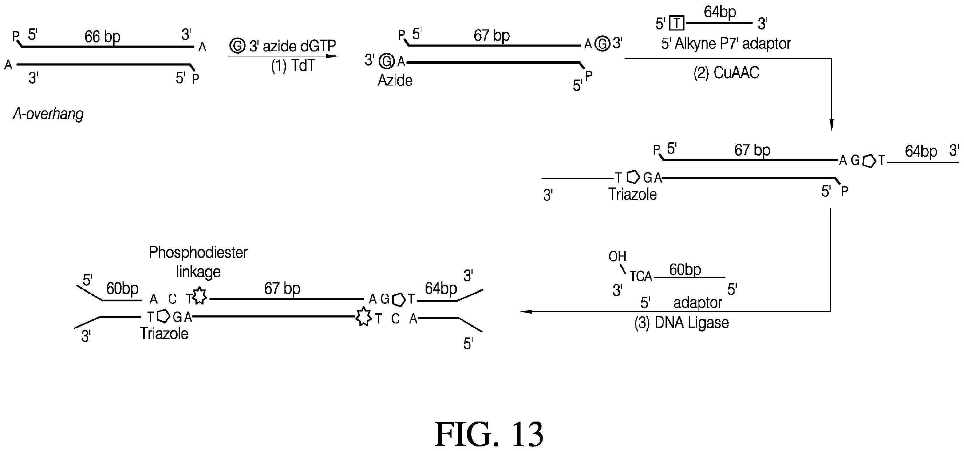

FIG. 13 illustrates a schematic of one exemplary method of preparing a library using the methods described herein using 3'-azido and 5'-alkyne.

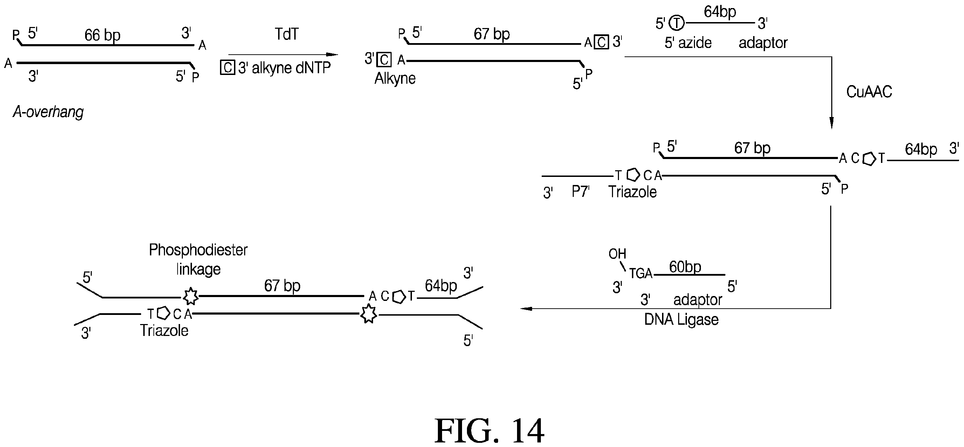

FIG. 14 illustrates a schematic of one exemplary method of preparing a library using the methods described herein using 3'-alkyne and 5'-azido.
FIGs. 15A-15B illustrate copper catalyzed azide-alkyne cycloaddition (CuAAC) of a natural polynucleotide and an adaptor with and without a splint where FIG. 15A shows the general reaction scheme and FIG. 15B shows a TBU PAGE analysis of the ligation.
FIGs. 16A-16D illustrate CuAAC of two polynucleotides with ligation and extension using various ligases and Klenow fragment. FIG. 16A shows the general scheme of ligation and extension. FIG. 16B illustrates TBU PAGE analysis of ligation. FIG. 16C shows ligation efficiency using T4 ligase (89.7% yield). FIG. 16D shows ligation efficiency using E. coli ligase (41.2% yield).

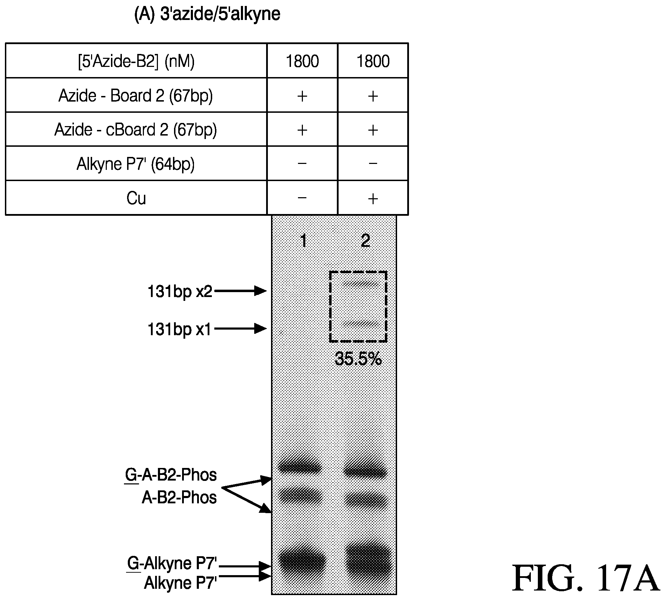

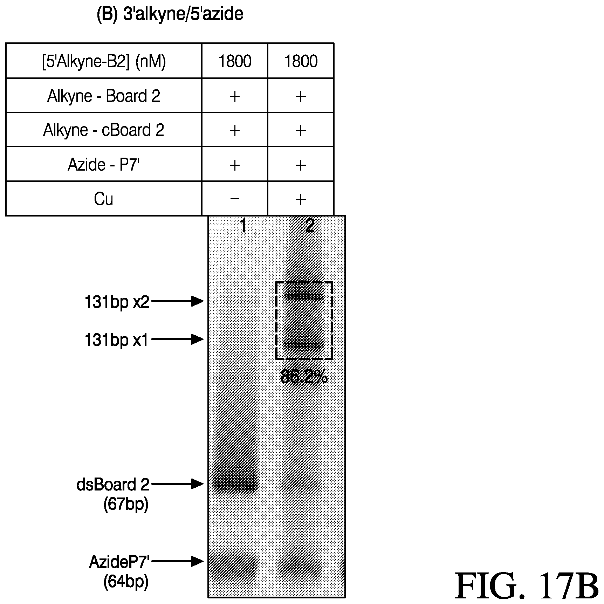

FIGs. 17A-17B illustrate click chemistry efficiency using a 3'-azido group for the polynucleotide input (FIG. 17A) and a 3'-alkynyl group for the polynucleotide fragment (FIG. 17B). The 3'-alkyne provided higher yield (86% compared to 36%) at the click chemistry step; a higher yield at the ligation step (72% compared to 12%) and an overall higher yield (62% compared to 4%).

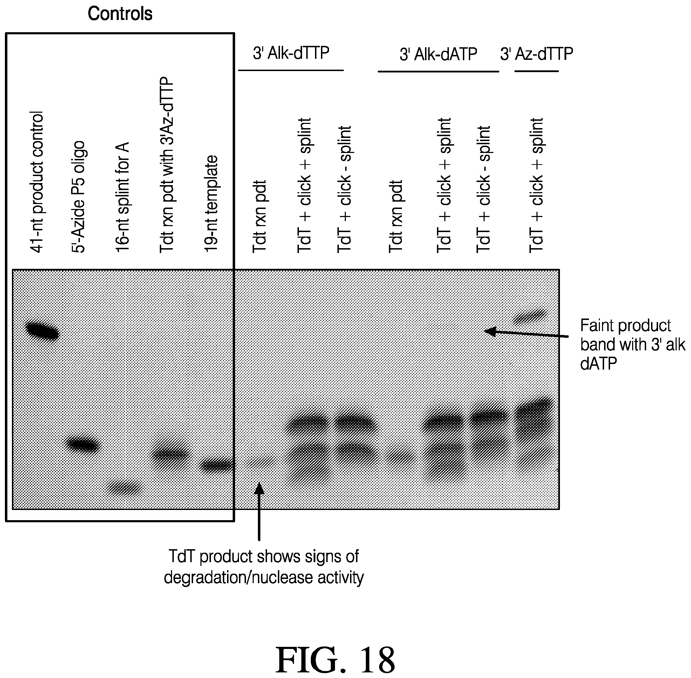

FIG. 18 illustrates presence of ligated product by click chemistry using gel analysis when performing the click reaction with a 3'-alkynyl input DNA sample.

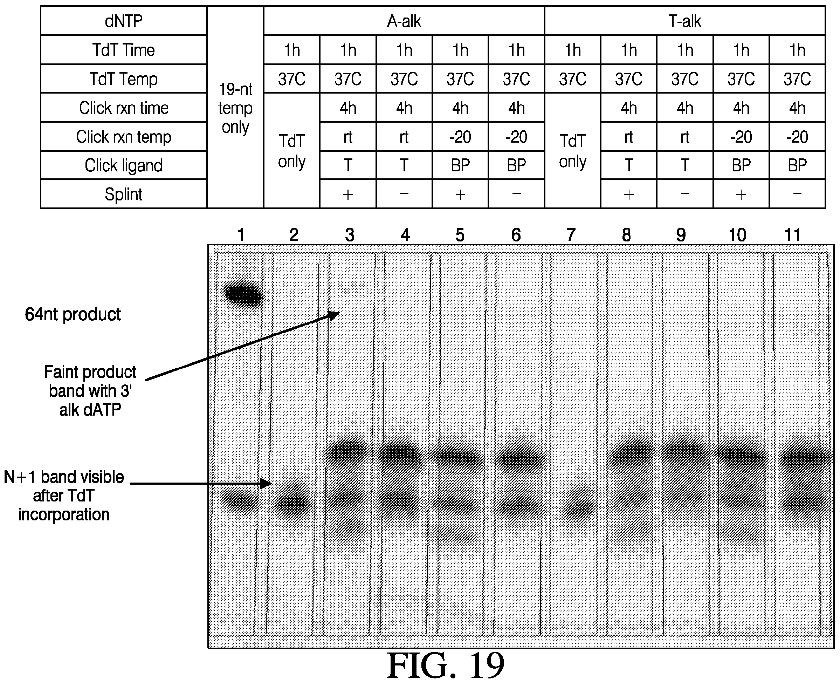

FIG. 19 illustrates testing of various conditions for ligation with input DNA containing a 3'-alkynyl.

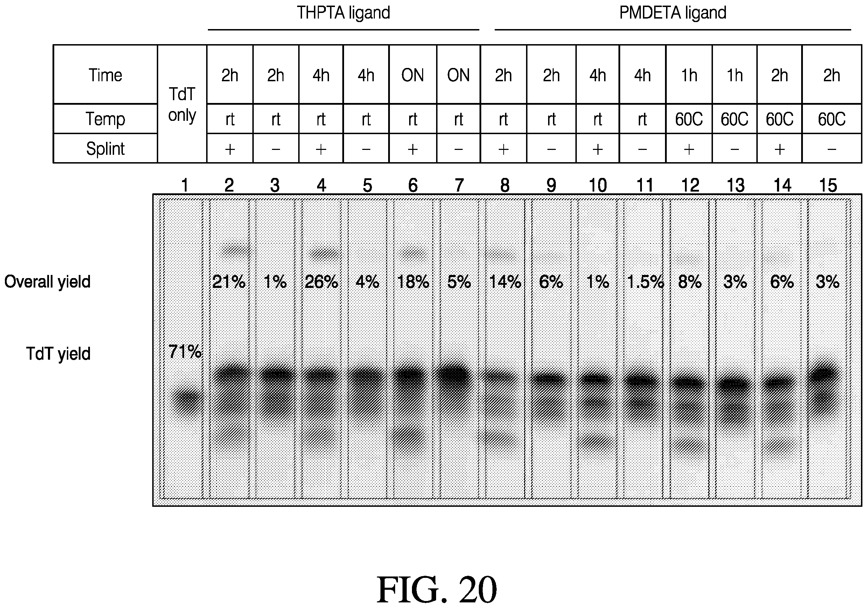

FIG. 20 illustrates ligation efficiency at various times and temperatures for performing the click chemistry reaction with a 3-azido ddTTP moiety in the input DNA. THPTA and PMDETA ligands were used in the reaction.

### DETAILED DESCRIPTION

This disclosure is directed to methods for synthesizing libraries of polynucleotides and capturing nucleic acids using chemical ligation techniques. The synthesis of libraries from a sample comprising cellular nucleic acids can be useful in a variety of applications where it is desirable to determine the nucleic acid sequence of a polynucleotide in the sample. In certain instances samples can comprise very little or presently undetectable amounts of polynucleotides. The methods described herein can be useful for capturing and sequencing of nucleic acid sequences from a sample of cellular nucleic acids.

Unless defined otherwise, all technical and scientific terms used herein have the same meaning as commonly understood by those of ordinary skill in the art to which this disclosure belongs. Any methods, devices and materials described herein can be used in the practice of the compositions and methods described herein. The following definitions are provided to facilitate understanding of certain terms used frequently herein and are not meant to limit the scope of the present disclosure.

Headings used in this application are for reference purposes only and do not in any way limit the present invention.

As used herein, and unless otherwise specified, the term "solid support" or other grammatical equivalents refers to any material that contains and/or can be modified to contain one or more sites (*e.g.*, discrete individual sites, pre-defined sites, random sites, etc.) appropriate for the attachment or association of compositions *(e.g.,* an organic molecule or moiety) disclosed herein. Exemplary solid supports include, but are not limited to, glass and modified or functionalized glass, plastics (including acrylics, polystyrene and copolymers of styrene and other materials, polypropylene, polyethylene, polybutylene, polyurethanes, Teflon, etc.), polysaccharides, nylon or nitrocellulose, resins, silica or silica-based materials including silicon and modified silicon, carbon, metals, inorganic glasses, optical fiber bundles, nanoparticles (e.g. inorganic nanoparticles (NPs) of cerium oxide (CeO₂), iron oxide (Fe₃O₄) and titanium oxide (TiO₂); or organic and bioorganic nanoparticles of lipids, nanocages, dendrimers, supermolecular nanoparticles, self-assembly nanoparticles), and a variety of other polymers. In particular embodiments, the solid supports allow optical detection and do not themselves appreciably fluoresce. Polynucleotides and nucleic acids described herein can be attached to the macromolecule directly or via a linker. As used herein, and unless otherwise specified, the term "linker" refers to the molecular fragment or moiety that joins the solid support and a polynucleotide.

A solid support can be flat (planar), although as will be appreciated by those in the art, other configurations of solid supports may be used as well; for example, three dimensional configurations can be used, for example by embedding beads in a porous block of plastic that allows sample access to the beads and using a confocal microscope for detection. Similarly, the beads may be placed on the inside surface of a tube, for flow-through sample analysis to minimize sample volume. In some aspects solid supports include optical fiber bundles and flat planar solid supports such as glass, polystyrene and other plastics and acrylics. A bead includes a small discrete particle, the composition of which will depend on the class of probe used and the method of synthesis. Suitable bead compositions include those used in peptide, nucleic acid and organic moiety synthesis, including, but not limited to, plastics, ceramics, glass, polystyrene, methylstyrene, acrylic polymers, paramagnetic materials, thoria sol, carbon graphite, titanium dioxide, latex or cross-linked dextrans such as Sepharose, cellulose, nylon, cross-linked micelles and Teflon may all be used. *See, e.g.,* "Microsphere Detection Guide" from Bangs Laboratories, Fishers IN.

In some embodiments, the solid support comprises one or more surfaces of a flowcell. The term "flowcell" as used herein refers to a chamber comprising a solid surface across which one or more fluid reagents can be flowed. Examples of flowcells and related fluidic systems and detection platforms that can be readily used in the methods of the present disclosure are described, for example, in Bentley et al., Nature 456:53-59 (2008), WO 04/018497; US 7,057,026; WO 91/06678; WO 07/123744; US 7,329,492; US 7,211,414; US 7,315,019; US 7,405,281, and US 2008/0108082, .

In some embodiments, the solid support comprises an array of wells or depressions in a surface. This may be fabricated as is generally known in the art using a variety of techniques, including, but not limited to, photolithography, stamping techniques, molding techniques and microetching techniques. As will be appreciated by those in the art, the technique used will depend on the composition and shape of the array substrate.

A "molecular tether" as used herein refers to a moiety covalently attached to a function moiety capable of participating in a click chemistry or chemical ligation reaction with a nucleic acid. A molecular tether can be a solid support as described above. A molecular tether can be a tag (e.g. a compound or other chemical molecule; nucleic acid; antibody, or peptide). When the molecular tether is a tag, the tag can be used to immobilize the nucleic acid to which it is attached to, for example, a solid support as described herein.

As used herein, the term "immobilized" when used in reference to a polynucleotide is intended to mean direct or indirect attachment to a solid support via covalent bond(s). In certain embodiments of the invention, covalent attachment can be used, but all that is required is that the polynucleotides remain stationary or attached to a support under conditions in which it is intended to use the support, for example, in applications requiring nucleic acid amplification and/or sequencing. Polynucleotides to be used as capture primers or amplification primers can be immobilized such that a 3'-end is available for enzymatic extension and/or modification and at least a portion of the sequence is capable of hybridizing to a complementary sequence. Immobilization can occur via hybridization to a surface attached oligonucleotide, in which case the immobilized oligonucleotide or polynucleotide can be in the 3' -5' orientation. Alternatively, immobilization can occur using the methods described herein.

As used herein, the terms "nucleic acid" and "nucleotide" are intended to be consistent with their use in the art and to include naturally occurring species or functional analogs thereof. Particularly useful functional analogs of nucleic acids are capable of hybridizing to a nucleic acid in a sequence specific fashion or capable of being used as a template for replication of a particular nucleotide sequence. Naturally occurring nucleic acids generally have a backbone containing phosphodiester bonds. An analog structure can have an alternate backbone linkage (e.g. a modified backbond linkage) including any of a variety of those known in the art. A "modified backbone linkage" as used herein refers to any bond(s) between adjacent bases in a polynucleotide that is not a phosphodiester bond. Exemplary modified backbone linkages include, but are not limited to: triazolyls; phosphorothioamidates; phsphoramidates; and phosphorothioates.

Naturally occurring nucleic acids ("Cellular Nucleic Acids") generally have a deoxyribose sugar *(e.g.,* found in deoxyribonucleic acid (DNA)) or a ribose sugar *(e.g.,* found in ribonucleic acid (RNA)). A nucleic acid can contain nucleotides having any of a variety of analogs of these sugar moieties that are known in the art. A nucleic acid can include native or non-native nucleotides. In this regard, a native deoxyribonucleic acid can have one or more bases selected from the group consisting of adenine, thymine, cytosine or guanine and a ribonucleic acid can have one or more bases selected from the group consisting of uracil, adenine, cytosine or guanine. Useful non-native bases that can be included in a nucleic acid or nucleotide are known in the art. A "dideoxynucleotide" or "ddNTP" refers to a nucleotide which does not have a 3' hydroxyl group. ddNTPs can have one or more bases selected from group consisting of adenine, thymine, uracil, cytosine or guanine.

As used herein, the term "polynucleotide" is intended to mean a ribonucleic or deoxyribonucleic acid or analog thereof, including a polynucleotide analyte presented in any context; for example, a probe, target or primer. Particular forms of polynucleotides of the invention include all types of nucleic acids found in an organism as well as synthetic nucleic acids such as polynucleotides produced by chemical synthesis. Particular examples of nucleic acids that are applicable for analysis through incorporation into microarrays produced by methods of the invention include genomic DNA (gDNA), DNA copied messenger RNA (cDNA), RNA copied messenger RNA (cRNA), mitochondrial DNA or genome, RNA, messenger RNA (mRNA) and/or other populations of RNA. Additional examples of polynucleotides include double stranded DNA (dsDNA), single stranded DNA (ssDNA), a gene or gene fragment (for example, a probe, primer, expressed sequence tag (EST) or serial analysis of gene expression (SAGE) tag), genomic DNA, exon, intron, transfer RNA (tRNA), ribosomal RNA (rRNA), ribozyme, recombinant polynucleotide, synthetic polynucleotide, branched polynucleotide, plasmid, vector, isolated DNA of any sequence, isolated RNA of any sequence or amplified copy of any of the foregoing. Fragments and/or portions of the above exemplary nucleic acids also are included within the meaning of the term as it is used herein unless otherwise described.

The terms "nucleic acid," "polynucleotide" and "oligonucleotide" are used interchangeably herein. The different terms are not intended to denote any particular difference in size, sequence, or other property unless specifically indicated otherwise.

As used herein, the term "double-stranded," when used in reference to a polynucleotide, means that some or all of the nucleotides between complementary strands of a polynucleotide are hydrogen bonded together to form a partial or complete double helix. A partially double stranded polynucleotide can have at least 10%, 25%, 50%, 60%, 70%, 80%, 90% or 95% of its nucleotides hydrogen bonded to a complementary nucleotide.

A single-stranded polynucleotide refers to a polynucleotide that has few to none hydrogen bonds with another polynucleotide such that a double helix is not formed or is unstable under a given set of hybridization conditions.

The term "modified" and the like, as used herein refer to a nucleotide or polynucleotide that has been chemically altered such that it includes a base comprising a functional moiety capable of participating in a click chemistry reaction. A 3'- modified moiety as described herein refers to modification at the 3' end of a nucleotide or polynucleotide. A 5'-modified moiety as described herein refers to modification at the 5' end of a nucleotide or polynucleotide.

A "ligated-nucleic acid" as used here refers to two or more nucleic acids covalently attached together using a click chemistry reaction described herein. In some embodiments, a ligated-nucleic acid includes a modified backbone linkage as described herein. A "ligated-cellular nucleic acid" as used herein refers to a nucleic acid covalently attached to another moiety other than a nucleotide using a click chemistry reaction described herein.

As used herein, the term "library," when used in reference to nucleic acids, is intended to mean a collection of nucleic acids having different chemical compositions (*e.g.*, different sequence, different length, etc.). Typically, the nucleic acids in a library will be different species having a common feature or characteristic of a genus or class, but otherwise differing in some way. For example, a library can include nucleic acid species that differ in nucleotide sequence, but that are similar with respect to having a sugar-phosphate backbone. A library can be created using techniques known in the art. Nucleic acids exemplified herein can include nucleic acids obtained from any source, including for example, digestion of a genome *(e.g.,* a human genome) or a mixture of genomes. In another example, nucleic acids can be those obtained from metagenomic studies of a particular environment or ecosystem. The term also includes artificially created nucleic acid libraries such as DNA libraries. In certain instances, such artificially created libraries can be useful for encoding information using DNA as exemplified by Church et al. Science 28 September 2012: Vol. 337 no. 6102 pp. 1628. A library of other target analytes having properties similar to those exemplified for nucleic acids can be useful as well.

As used herein, the term "capture primer" is intended to mean an oligonucleotide having a nucleotide sequence that is capable of specifically annealing to a single stranded polynucleotide sequence to be analyzed or subjected to a nucleic acid interrogation under conditions encountered in a primer annealing step of, for example, an amplification or sequencing reaction. The term also is intended to mean an oligonucleotide having a nucleotide sequence that is capable of specifically annealing to a single stranded polynucleotide sequence that is used to analyze, interrogate or perform an action on another molecular entity.

A capture primer can include one or more capture regions. A capture primer region can include, for example, a universal capture region, a sequencing primer binding site (SpBS), a target-specific capture region, a predetermined cleavage site, such as a restriction site, and a linker region, for example, a linker region separating two or more regions of the capture primer. Some capture primers can include, for example, a universal capture region and a SpBS. Other capture primers can include a universal capture region and a target-specific capture region. Still other capture primers can include, for example, a universal capture region, a SpBS and a target-specific region. A capture primer can be blocked at the 3'-end (3'-blocked) or unblocked at the 3'-end (3'-unblocked). A primer with a blocked 3'-end can, for example, can be deblocked in an enzymatic or chemical reaction. A capture primer also can include a predetermined (nonrandom) cleavage site. Exemplary predetermined cleavage sites are described, for example, in U.S. Patent No. 8,715,966, which is incorporated herein by reference in its entirety and for all purposes. Cleavage at predetermined sites can occur, for example, as enzymatic cleavage or non-enzymatic cleavage, such as chemical cleavage using methods well known to those skilled in the art. Given the teachings and guidance provided herein, those skilled in the art will understand that a capture primer can contain any number of different regions that are useful in one or more applications.

In comparison, the term "universal" when used in reference to a capture primer or other oligonucleotide sequence is intended to mean a capture primer or other oligonucleotide having a common nucleotide sequence among a plurality of capture primers. A common sequence can be, for example, a sequence complementary to the same adapter sequence. Universal capture primers are applicable for interrogating a plurality of different polynucleotides without necessarily distinguishing the different species whereas target specific capture primers are applicable for distinguishing the different species.

"Restriction endonuclease" is intended to be consistent with its use in the art and refers to an enzyme that after binding to its recognition sequence (e.g. "Restriction Site"), hydrolyzes both strands of duplex polynucleotide.

"Nicking endonuclease" is intended to be consistent with their use in the art and refers to an enzyme that after binding to its recognition sequence hydrolyzes only one of the strands of duplex polynucleotide.

A "template independent polymerase" refers to an enzyme that catalyzes addition of one or more nucleotides into/onto a polynucleotide without a template strand. A "RNA specific nucleotidyl transferase" as used herein refers to an enzyme that polymerizes ribonucleotides at the 3' end of a RNA-polynucleotide. A DNA-specific nucleotidyl transferase" as used herein refers to an enzyme that polymerizes deoxynucleotides at the 3' end of a DNA-polynucleotide.

As used herein, the term "each," when used in reference to a collection of items, is intended to identify an individual item in the collection but does not necessarily refer to every item in the collection. Exceptions can occur if explicit disclosure or context clearly dictates otherwise.

The terms "click chemistry" and "click chemistry reaction" are used interchangeably herein and are intended to be consistent with their use in the art. Generally click chemistry reactions are fast (e.g. quick to completion of reaction), simple, easily purified, and regiospecific. Click chemistry includes reactions such as, but not limited to, copper catalyzed azide-alkyne cycloaddition (CuAAC); strain-promoted azide-alkyne cycloaddition (SPAAC) also known as copper-free click chemistry; strain-promoted alkyne-nitrone cycloaddition (SPANC); alkyne hydrothiolation; and alkene hydrothiolation. Click chemistry using Cu as a catalyst often includes a Cu stabilizing ligand that is labile. Without being bound by any particular theory, the ligand can stabilize/protect the Cu ion from oxidizing from the reactive Cu(I) species to the Cu(II) species and can also act as a proton acceptor reducing or eliminating requirement of a base in the reaction.

Click chemistry between polynucleotides can in some embodiments, be assisted by using a moiety that brings the two reacting partners in close enough proximity to react. In some embodiments herein, ligated-polynucleotides described herein can be synthesized using the methods herein in the presence of a splint. A "splint" refers to a short-length polynucleotide having complementary sequence to the region where the click chemistry reaction will occur between the two reacting polynucleotides. In some embodiments, the splint is 5, 6, 7, 8, 9, 10, 15, 20, 25, 35, 40, 45, 50 or more nucleotides in size.

As used herein, and unless otherwise indicated, the term "adding," "reacting," "treating," or the like means contacting one reactant, reagent, solvent, catalyst, reactive group or the like with another reactant, reagent, solvent, catalyst, reactive group or the like. Reactants, reagents, solvents, catalysts, reactive groups or the like can be added individually, simultaneously or separately and can be added in any order that achieves a desired result. They can be added in the presence or absence of a heating or cooling apparatus and can optionally be added under an inert atmosphere.

As used herein, and unless otherwise specified, the term "alkyl" refers to a linear or branched saturated hydrocarbon radical. The term "alkyl" also encompasses both linear and branched alkyl, unless otherwise specified. In certain embodiments, the alkyl is a linear saturated hydrocarbon radical that has 1 to 6 (C₁₋₆) or 1 to 3 (C₁₋₃) carbon atoms, or branched saturated hydrocarbon radical of 3 to 6 (C₃₋₆) carbon atoms. Examples of alkyl groups include, but are not limited to, methyl, ethyl, propyl (including all isomeric forms), n-propyl, isopropyl, butyl (including all isomeric forms), n-butyl, isobutyl, t-butyl, pentyl (including all isomeric forms), and hexyl (including all isomeric forms). The alkyl can be unsubstituted or substituted with one or more substituents. As used herein, the alkyl can be either a monovalent radical, or a multivalent radical *(e.g.,* an alkylene) when it is described to be attached to more than one groups or moieties.

As used herein, and unless otherwise specified, the term "alkenyl" refers to a linear or branched hydrocarbon radical, which contains one or more, in one embodiment, one to five, carbon-carbon double bonds. The term "alkenyl" also embraces radicals having "*cis*" and *"trans"* configurations, or alternatively, "E" and "Z" configurations, as appreciated by those of ordinary skill in the art. As used herein, the term "alkenyl" encompasses both linear and branched alkenyl, unless otherwise specified. For example, C₂₋₆ alkenyl refers to a linear unsaturated hydrocarbon radical of 2 to 6 carbon atoms or a branched unsaturated hydrocarbon radical of 3 to 6 carbon atoms. In certain embodiments, the alkenyl is a linear hydrocarbon radical of 2 to 6 (C₂₋₆) or 2 to 3 (C₂₋₃) carbon atoms, or a branched hydrocarbon radical of 3 to 10 (C₃₋₁₀) or 3 to 6 (C₃₋₆) carbon atoms. Examples of alkenyl groups include, but are not limited to, ethenyl, propen-1-yl, propen-2-yl, allyl, butenyl, and 4-methylbutenyl. The alkenyl can be unsubstituted or substituted with one or more substituents. As used herein, the alkenyl can be either a monovalent radical, or a multivalent radical *(e.g.,* an alkenylene) when it is described to be attached to more than one groups or moieties.

As used herein, and unless otherwise specified, the term "alkynyl" refers to a linear or branched hydrocarbon radical, which contains one or more, in one embodiment, one to five, carbon-carbon triple bonds. The term "alkynyl" also encompasses both linear and branched alkynyl, unless otherwise specified. In certain embodiments, the alkynyl is a linear hydrocarbon radical of 2 to 6 (C₂₋₆) or 2 to 3 (C₂₋₃) carbon atoms, or a branched hydrocarbon radical of 3 to 10 (C₃₋₁₀) or 3 to 6 (C₃₋₆) carbon atoms. Examples of alkynyl groups include, but are not limited to, ethynyl (-C≡CH) and propargyl (-CH₂C≡CH). For example, C₂₋₆ alkynyl refers to a linear unsaturated hydrocarbon radical of 2 to 6 carbon atoms or a branched unsaturated hydrocarbon radical of 3 to 6 carbon atoms. The alkynyl can be unsubstituted or substituted with one or more substituents. As used herein, the alkynyl can be either a monovalent radical, or a multivalent radical *(e.g.,* an alkynylene) when it is described to be attached to more than one groups or moieties.

As used herein, and unless otherwise specified, the term "amino" refers to - N(R°)(R°), wherein each R° independently can be, but is not limited to, hydrogen, alkyl, heteroalkyl, alkenyl, alkynyl, aryl, cycloalkyl, heteroaryl, heterocyclyl, each of which is defined above. When a -N(R°)(R°) group has two R° other than hydrogen, they can be combined with the nitrogen atom to form a ring. In one embodiment, the ring is a 3-, 4-, 5-, 6-, 7-, or 8-membered ring. In one embodiment, one or more ring atoms are heteroatoms independently selected from O, S, or N. As used herein, and unless otherwise specified, the term "primary amino" refers to -NH₂. The term "amino" also includes N-oxide -N⁺(R°)(R°)O⁻. In certain embodiments, each R° or the ring formed by -N(R°)(R°) independently may be unsubstituted or substituted with one or more substituents.

As used herein, and unless otherwise specified, the term "amide" or "amido" refers to -C(O)N(R°)₂ or -NR°C(O)R°, wherein each R° independently can be, but is not limited to, hydrogen, alkyl, heteroalkyl, alkenyl, alkynyl, aryl, cycloalkyl, heteroaryl, heterocyclyl, each of which is defined above. When a -C(O)N(R°)₂ group has two R° other than hydrogen, they can be combined with the nitrogen atom to form a ring. In one embodiment, the ring is a 3-, 4-, 5-, 6-, 7-, or 8-membered ring. In one embodiment, one or more ring atoms are heteroatoms independently selected from O, S, or N. In certain embodiments, each R° or the ring formed by -N(R°)(R°) independently may be unsubstituted or substituted with one or more substituents.

As used herein, and unless otherwise specified, the term "azide" or "azido" refers to -N⁻= N⁺≡N.

As used herein, and unless otherwise specified, the term "nitrone" refers to 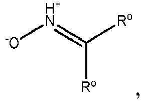 wherein each R° independently can be, but is not limited to, hydrogen, alkyl, heteroalkyl, alkenyl, alkynyl, aryl, cycloalkyl, heteroaryl, heterocyclyl, each of which is defined above. In certain embodiments, each R° is hydrogen.

As used herein, and unless otherwise specified, the term "phosphorothioamidate" and the like refers to 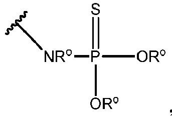 , wherein each R° independently can be, but is not limited to, hydrogen, alkyl, heteroalkyl, alkenyl, alkynyl, aryl, cycloalkyl, heteroaryl, heterocyclyl, each of which is defined above. In certain embodiments, each R° is hydrogen.

As used herein, and unless otherwise specified, the term "phosphoroamidate" and the like refers to 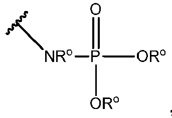 , wherein each R° independently can be, but is not limited to, hydrogen, alkyl, heteroalkyl, alkenyl, alkynyl, aryl, cycloalkyl, heteroaryl, heterocyclyl, each of which is defined above. In certain embodiments, each R° is hydrogen.

As used herein, and unless otherwise specified, the term "phosphorothioate" and the like refers to 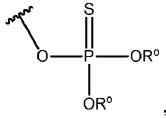 , wherein each R° independently can be, but is not limited to, hydrogen, alkyl, heteroalkyl, alkenyl, alkynyl, aryl, cycloalkyl, heteroaryl, heterocyclyl, each of which is defined above. In certain embodiments, each R° is hydrogen.

As used herein, and unless otherwise specified, the term "sulfanyl", "sulfide", or "thio" refers to -S-R^{t}, wherein R' can be, but is not limited to, alkyl, heteroalkyl, alkenyl, alkynyl, aryl, cycloalkyl, heteroaryl, heterocyclyl, each of which is defined above. In certain embodiments, R^{t} may be unsubstituted or substituted with one or more substituents.

As used herein, and unless otherwise specified, the term "sulfoxide" refers to - S(O)-R^{t}, wherein R' can be, but is not limited to, alkyl, heteroalkyl, alkenyl, alkynyl, aryl, cycloalkyl, heteroaryl, heterocyclyl, each of which is defined above. In certain embodiments, R^{t} may be unsubstituted or substituted with one or more substituents.

As used herein, and unless otherwise specified, the term "sulfonyl" or "sulfone" refers to -S(O)₂-R^{t}, wherein R' can be, but is not limited to, alkyl, heteroalkyl, alkenyl, alkynyl, aryl, cycloalkyl, heteroaryl, heterocyclyl, each of which is defined above. In certain embodiments, R^{a} may be unsubstituted or substituted with one or more substituents.

As used herein, and unless otherwise specified, the term "sulfonamido" or "sulfonamide" refers to -S(=O)₂-N(R°)₂ or -N(R°)-S(=O)₂-R°, wherein each R° independently can be, but is not limited to, hydrogen, alkyl, heteroalkyl, alkenyl, alkynyl, aryl, cycloalkyl, heteroaryl, heterocyclyl, each of which is defined above. When a -S(=O)₂-N(R°)₂ group has two R° other than hydrogen, they can be combined with the nitrogen atom to form a ring. In one embodiment, the ring is a 3-, 4-, 5-, 6-, 7-, or 8-membered ring. In one embodiment, one or more ring atoms are heteroatoms independently selected from O, S, or N. In certain embodiments, each R° or the ring formed by -N(R°)(R°) independently may be unsubstituted or substituted with one or more substituents.

When the groups described herein are said to be "substituted," they may be substituted with any appropriate substituent or substituents. Illustrative examples of substituents include, but are not limited to, those found in the exemplary compounds and embodiments described herein, as well as halogen (chloro, iodo, bromo, or fluoro); alkyl; alkenyl; alkynyl; hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonate; phosphine; thiocarbonyl; sulfinyl; sulfone; sulfonamide; ketone; aldehyde; ester; urea; urethane; oxime; hydroxyl amine; alkoxyamine; aryloxyamine, aralkoxyamine; N-oxide; hydrazine; hydrazide; hydrazone; azide; isocyanate; isothiocyanate; cyanate; thiocyanate; oxo (=O); B(OH)₂, O(alkyl)aminocarbonyl. Other illustrative examples include cycloalkyl, which may be monocyclic or fused or non-fused polycyclic *(e.g.,* cyclopropyl, cyclobutyl, cyclopentyl, or cyclohexyl), or a heterocyclyl, which may be monocyclic or fused or non-fused polycyclic *(e.g.,* pyrrolidyl, piperidyl, piperazinyl, morpholinyl, or thiazinyl); monocyclic or fused or non-fused polycyclic aryl or heteroaryl (*e.g*., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridazinyl, pyrimidinyl, benzimidazolyl, benzothiophenyl, or benzofuranyl) aryloxy; aralkyloxy; heterocyclyloxy; and heterocyclyl alkoxy.

As used herein, and unless otherwise specified, the term "about" or "approximately" means an acceptable error for a particular value as determined by one of ordinary skill in the art, which depends in part on how the value is measured or determined. In certain embodiments, the term "about" or "approximately" means within 1, 2, 3, or 4 standard deviations. In certain embodiments, the term "about" or "approximately" means within 50%, 20%, 15%, 10%, 9%, 8%, 7%, 6%, 5%, 4%, 3%, 2%, 1%, 0.5%, or 0.05% of a given value or range.

In one aspect of the invention is a method of preparing a nucleic acid library from a sample, as defined in the appended claims. In one embodiment, the method comprises: (i) reacting a sample comprising a plurality of cellular nucleic acids each having a terminal 3'-modified dideoxynucleotide (ddNTP) comprising a 3'-functional moiety capable of participating in a click chemistry reaction, with a plurality of adaptor nucleic acids each comprising a terminal 5'-modified ddNTP comprising a compatible 5'-functional moiety capable of participating in a click chemistry reaction with the 3' functional moiety, wherein the 3' functional moiety and 5' functional moiety react to form a modified backbone linkage, thereby forming a plurality of ligated-nucleic acids, and (ii) amplifying the plurality of ligated-nucleic acids thereby preparing a nucleic acid library from the sample.

In another embodiment the method of preparing a nucleic acid library from a sample comprises (i) reacting a sample comprising a plurality of cellular nucleic acids each comprising a terminal 3'-modified ddNTP comprising a 3'-functional moiety capable of participating in a chemical ligation reaction, wherein the 3'-modified ddNTP is incorporated into each of the plurality of cellular nucleic acids by contacting the plurality of cellular nucleic acids with a template independent polymerase, with a plurality of adaptor nucleic acids each comprising a terminal 5'-modified ddNTP comprising compatible 5'-functional moiety capable of participating in a chemical ligation reaction with the 3' functional moiety, wherein the 3' functional moiety and 5' functional moiety react to form a modified backbone linkage, thereby forming a plurality of ligated-nucleic acids; and (ii) amplifying the plurality of ligated-nucleic acids thereby preparing a nucleic acid library from the sample.

In another embodiment, the method of preparing a nucleic acid library from a sample comprises: (i) reacting a sample comprising a plurality of cellular nucleic acids each having a terminal 3'-modified ddNTP comprising a 3'-functional moiety capable of participating in a click chemistry reaction, wherein the terminal 3'-modified ddNTP is incorporated into each of the plurality of cellular nucleic acids by contacting the plurality of cellular nucleic acids with a template independent polymerase with a plurality of adaptor nucleic acids attached to a support, wherein each of the plurality of adaptor nucleic acids comprises a terminal 5'-modified ddNTP comprising a compatible 5'-functional moiety capable of participating in a click chemistry reaction with the 3' functional moiety, wherein the 3' functional moiety and 5' functional moiety react to form a modified backbone linkage, thereby forming a plurality of ligated-nucleic acids, and (ii) amplifying the plurality of ligated-nucleic acids thereby preparing a nucleic acid library from the sample.

In certain embodiments the terminal 3'-modified ddNTP is incorporated into each of the plurality of cellular nucleic acids by contacting the plurality of cellular nucleic acids with a template independent polymerase. In one embodiment, the template independent polymerase can be a RNA-specific nucleotidyl transferase. Exemplary RNA-specific nucleotidyl transferases useful in the methods described herein include, but are not limited to, poly(A) polymerase polymerases, CCA-adding enzymes, terminal uridylyl transferases, and poly(U) polymerases. In another embodiment, the template independent polymerase is a DNA-specific nucleotidyl transferase. Exemplary DNA-specific nucleotidyl transferases include, but are not limited to, polymerase lambda (pol λ), polymerase mu (pol µ), and terminal deoxynucleotidyl transferase (TdT). In one embodiment, the template independent polymerase is selected from the group consisting of poly(A) polymerase polymerases, CCA-adding enzymes, terminal uridylyl transferases, poly(U) polymerases, and terminal deoxynucleotidyl transferase (TdT). In one embodiment, the template independent polymerase is terminal deoxynucleotidyl transferase (TdT), PolyA polymerase, or CCA-adding RNA polymerase. In a particular embodiment, the template independent polymerase is TdT.

In certain embodiments, the template independent polymerase is a mutant template independent polymerase (e.g. a template independent polymerase comprising one or more amino acid changes from the native sequence). Mutant template independent polymerases can include any number of mutations as understood by those skilled in the, so long as the mutant template independent polymerase retains the function of the native polypeptide sequence. In one embodiment, the mutant template independent polymerase has lower activity but better stability (e.g. greater stability at higher or lower temperature than the native template independent polymerase). In one embodiment, the mutant template independent polymerase has at least equivalent activity to the native template independent polymerase. In still another embodiment, the mutant template independent polymerase has greater activity than the native template independent polymerase. In yet another embodiment, the mutant template independent polymerase has equivalent or better stability and equivalent or better activity than the native template independent polymerase.

The template independent polymerase can incorporate the terminal 3'-modified ddNTP at a yield of at least 70%, 75%, 80%, 85%, 90%, 95%, or more (e.g. as related to the percent of the entire number of sequences or related to a given species). In one embodiment, the template independent polymerase can incorporate the terminal 3'-modified ddNTP at a yield of at least 25%, 30%, 40%, 50%, 60%, 70%, or more

In certain embodiments, the template independent polymerase is present at an amount of at least 0.05 µM, 0.1 µM, 0.2 µM, 0.3 µM, 0.4 µM,.5 µM, 1 µM, 2 µM, 5 µM, 7 µM, 10 µM, or more. In other embodiments, the template independent polymerase is present at an amount of at least 0.05 µM to at least 50 µM, 0.05 µM to at least 40 µM, 0.05 µM to at least 30 µM, 0.05 µM to at least 25 µM, 0.05 µM to at least 20 µM, 0.05 µM to at least 10 µM, or 0.05 µM to at least 5 µM. In other embodiments, the template independent polymerase is present at an amount of at least 0.5 µM to at least 50 µM, 0.5 µM to at least 40 µM, 0.5 µM to at least 30 µM, 0.5 µM to at least 25 µM, 0.5 µM to at least 20 µM, 0.5 µM to at least 10 µM, or 0.5 µM to at least 5 µM. In other embodiments, the template independent polymerase is present at an amount of at least 1 µM to at least 50 µM, at least 5 µM to at least 50 µM, or at least 10 µM to at least 50 µM.

In one embodiment, the terminal 3'-modified ddNTP is incorporated by the template independent polymerase in a reaction performed at a temperature of about 20 °C to about 40 °C. In another embodiment, the terminal 3'-modified ddNTP is incorporated by the template independent polymerase in a reaction performed at a temperature of about 10, 20, 25, 30, 35, 40, 45, 50, or 55 °C. In another embodiment, the terminal 3'-modified ddNTP is incorporated by the template independent polymerase in a reaction performed for over a time of about 15 min, 20 min, 30 min 60 min, 90 min, 120 min, or more.

In one embodiment, TdT can incorporate the terminal 3'-modified ddNTP at a yield of at least 70%, 75%, 80%, 85%, 90%, 95%, or more (e.g. as related to the percent of the entire number of sequences or related to a given species). In one embodiment, TdT can incorporate the terminal 3'-modified ddNTP at a yield of at least 30% to 90%, 30% to 95%, or 30% to about 100%. In one embodiment, TdT can incorporate the terminal 3'-modified ddNTP at a yield of at least 25%, 30%, 40%, 50%, 60%, 70%, or more (e.g. as related to the percent of the entire number of sequences or related to a given species). In another embodiment, incorporate the terminal 3'-modified ddNTP at a yield of about 30%, 40%, 50%, 60%, 70%, 75%, 80%, 85%, 90%, 95%, or more (e.g. as related to the percent of the entire number of sequences or related to a given species).

In certain embodiments, TdT is present at an amount of at least 0.5 µM, 1 µM, 2 µM, 5 µM, 7 µM, 10 µM, or more. In other embodiments, TdT is present at an amount of at least 0.5 µM to at least 50 µM, 0.5 µM to at least 40 µM, 0.5 µM to at least 30 µM, 0.5 µM to at least 25 µM, 0.5 µM to at least 20 µM, 0.5 µM to at least 10 µM, or 0.5 µM to at least 5 µM. In other embodiments, TdT is present at an amount of at least 1 µM to at least 50 µM, at least 5 µM to at least 50 µM, or at least 10 µM to at least 50 µM.

The 3' functional moiety of the terminal 3'-modified ddNTP can be selected from the group consisting of an azide, alkynyl, alkenyl, a thiol, or a nitrone. In one embodiment, the 3' functional moiety of the terminal 3'-modified ddNTP can be an azide or alkynyl. In one embodiment, the 3' functional moiety of the terminal 3'-modified ddNTP can be an azide. In one embodiment, the 3' functional moiety of the terminal 3'-modified ddNTP can be an alkynl.

The 5' functional moiety of the terminal 5'-modified ddNTP can be selected from the group consisting of an azide, alkynyl, alkenyl, a thiol, or a nitrone. In one embodiment, the 5' functional moiety of the terminal 5'-modified ddNTP can be an azide or alkynyl. In one embodiment, the 5' functional moiety of the terminal 5'-modified ddNTP can be an azide. In one embodiment, the 5' functional moiety of the terminal 5'-modified ddNTP can be an alkynl.

In one embodiment, the 3' functional moiety and the 5' functional moiety are selected from the following pairs:
(i) 3'-azido / 5'- alkynyl;
(ii) 3'-alkynyl / 5' azido;
(iii) 3'-thiol / 5'-alkynyl;
(iv) 3'-thiol / 5'-alkenyl;
(v) 3'-alkynyl / 5'-thiol;
(vi) 3'-alkenyl / 5'-thiol;
(vii) 3'-azido / 5'-cyclooctynyl;
(viii) 3'-cyclooctyne / 5'-azido;
(ix) 3'-nitrone / 5'-cyclooctynyl; or
(x) 3'-cyclooctynyl / 5'-nitrone.

In one embodiment, the 3' functional moiety comprises a 3'-azido and the 5' functional moiety comprises a 5'-alkynyl (e.g. pair (i)). In another embodiment, the pair is pair (ii). In one embodiment, the 5' functional moiety is different from and compatible with the 3' functional moiety. In one embodiment, the 5' functional moiety is different from and compatible with the 3' functional moiety and is selected from the group consisting of an azide, alkynyl, alkenyl, a thiol, or a nitrone. In one embodiment, the 5' functional moiety is different from and incompatible with the 3' functional moiety, but can be compatible with a second 3' functional moiety.

In one embodiment, the 3' functional moiety of the terminal 3'-modified ddNTP can be incorporated by the template independent polymerase in less than 10, 20, 30, 60, 120 min. In another embodiment, the 3' functional moiety of the terminal 3'-modified ddNTP can be incorporated by the template independent polymerase can be incorporated in less than about 1, 2, 3, 4, 5, 6, 10, or 12 hours. In one embodiment, the 3' functional moiety of the terminal 3'-modified ddNTP can be incorporated by the template independent polymerase at a rate equal to the efficiency of incorporating the corresponding natural ddNTP. In one embodiment, the 3' functional moiety of the terminal 3'-modified ddNTP can be incorporated by the template independent polymerase at a rate greater than the efficiency of incorporating the corresponding natural ddNTP.

Click chemistry used in the methods described includes all known types and variations of performing click chemistry as known and understood to those skilled in the art. For example, click chemistry useful in the methods described herein can comprise copper catalyzed azide-alkyne cycloaddition (CuAAC), forming a modified backbone linkage comprising a triazolyl. When CuAAC is used in the methods described herein the CuAAC reaction can further include a Cu(I) stabilizing ligand. CuAAC ligands are well known in the art and employ various types of moieties and heteroatoms.

In one embodiment, the Cu(I) stabilizing ligand is selected from the group consisting of: 3-[4-({bis[(1-*tert*-butyl-1*H*-1,2,3-triazol-4-yl)methyl]amino}methyl)-1*H*-1,2,3-triazol-1-yl]propanol (BTTP); 3-[4-({bis[(1-*tert*-butyl-1*H*-1,2,3-triazol-4-yl)methyl]amino}methyl)-1*H-*1,2,3-triazol-1-yl]propyl hydrogen sulfate (BTTPS); 2-[4-({bis[(1-tert-butyl-1H-1,2,3-triazol-4-yl)methyl]amino}methyl)-1H-1,2,3-triazol-1-yl]ethyl hydrogen sulfate (BTTES); bathophenanthroline disulphonate disodium salt (BTTAA); N^{ε}-((1R,2R)-2-azidocyclopentyloxy)carbonyl)-L-lysine (BPS); pentamethyldiethylenetriamine (PMDETA); tris(2-benzimidazolylmethyl)amine ((BimH)₃) tris-(benzyltriazolylmethyl)amine (TBTA); and tris(3-hydroxypropyltriazolylmethyl)amine (THPTA). In another embodiment, the Cu(I) stabilizing ligand can be TBTA, PMDETA, or THPTA. In still another embodiment, the Cu(I) stabilizing ligand can be TBTA or THPTA.

The click chemistry reaction can comprise strain-promoted azide-alkyne cycloaddition (SPAAC) to form a modified backbone linkage comprising a cycloocta-triazolyl. In one embodiment, click chemistry reaction comprises alkyne hydrothiolation to form a modified backbone linkage comprising an alkenyl sulfide. In another embodiment, click chemistry reaction comprises alkene hydrothiolation to form a modified backbone linkage comprising an alkyl sulfide. In another embodiment, click chemistry reaction comprises strain-promoted alkyne-nitrone cycloaddition (SPANC) to form a modified backbone linkage comprising an octahydroocycloocta-isoxazolyl. The cyclooctynyl can be any compound known in the art useful in such types of click chemistry reactions. In one embodiment, the cyclooctynyl is dibenzylcyclooctyne (DBCO) or a derivative thereof. In one embodiment, the cyclooctynyl is DBCO. Various cyclooctynyl derivatives are known and useful in the art. For example, DBCO derivative can include functional moieties such as esters, amines, maleimides, alcohols, acids, and conjugates (e.g. biotin/avidin). In other examples, DBCO derivatives can include linkers and reporting molecules. In certain embodiments, SPAAC can be used to make libraries described herein, wherein the modified backbone of the ligated-nucleic acids can be read-through by an enzyme (e.g. a polymerase).

In one embodiment, the click chemistry reaction has a greater yield of ligated-nucleic acids than compared to enzyme-ligated nucleic acids. The yield can be 1%, 5%, 10%, 20%, 50%, 100% or greater percent when using the methods described herein compared to enzyme-ligated nucleic acids. The yield can be 0.1-, 0.5-, 1-, 2-, 5-, 10-, 20-, 50-fold greater when using the methods described herein compared to enzyme-ligated nucleic acids.

In one embodiment, the click chemistry reaction yields at least 70%, 75%, 80%, 85%, 90%, 95%, or more ligated-nucleic acids. In one embodiment, the click chemistry reaction yields at least 25%, 30%, 40%, 50%, 60%, 65%, or more ligated-nucleic acids. The percent of ligated-nucleic acids can be a measure based upon, for example, concentration, or for example, successful reads of the polynucleotides at the time of sequencing.

Click chemistry reactions described herein can be biocompatible. In certain embodiments, the click chemistry reactions described herein can be performed at a predetermined temperature. That is, click chemistry reactions described herein can be performed at a temperature of about 20 °C to about 40 °C; about 20 °C to about 65 °C; about 20 °C to about 75 °C; about 10 °C to about 60 °C; about, or about 10 °C to about 40 °C.

The click chemistry reactions described herein were also surprisingly found to be efficient at low temperatures - e.g. temperatures considered lower than those for viable enzymatic ligation. In one embodiment, the click chemistry reactions described herein can be performed at a temperature less than 0 °C. In certain embodiments, the click chemistry reaction can be performed at a temperature of about -4 °C to about -20 °C.

The time of the click chemistry reaction can also increase the efficiency of the formation of ligated-nucleic acids. For example, longer times can in some embodiments, provide sufficient time for reactions to complete but can in other embodiments decrease yield as a result of other factors such as product/reactant degradation. The click chemistry reactions described herein can be performed at times as short as about 10 min to times greater than about 5, 10, 16, or 24 hours. In one embodiment, the click chemistry reaction is performed for 10 min, 20 min, 30 min, 1 hr, 2 hr, 3 hr, 4 hr, or more.

Click chemistry reaction efficiency can also be dependent on the relative concentrations and kinetics of the reactants. Without being bound by particular theories, by scaffolding or "splinting" the polynucleotides independently comprising the 3' functional moiety and the 5' functional moiety, the reaction efficiency can be increased. In one embodiment, a click chemistry reaction useful in the methods described herein includes a splint comprising a polynucleotide sequence complementary to the site of click chemistry. The splint can be about 10, 15, 20, 25, 30, 40 or 50 nucleotides in length. In another embodiment, the splint can be about 5 to about 50; about 5 to about 30; about 5 to about 25; about 5 to about 20, about 5 to about 10; about 10 to about 30; or about 20 to about 40 nucleotides in length.

In certain embodiments, it can be desirable to have a modified backbone linkage for which a template independent or template dependent polymerase can read-through. It is known in the art, for example, that CuAAC linked polynucleotides having a triazolyl moiety for a modified backbone linkage have permissible read-through by polymerases. In particular, when the methods described herein are used to amplify a ligated-nucleic acid having a modified backbone linkage it can particularly advantageous that a polymerase be able to read-through the ligated-nucleic acid. In certain embodiments, the plurality of ligated-nucleic acids can be amplified by a polymerase.

In another embodiment, linkages may be useful for immobilizing nucleic acids described herein where amplification may not be needed. In certain embodiments, where amplification is not needed, the method includes an additional click chemistry reaction to ligate two or more polynucleotides using a linkage that cannot be amplified or otherwise stops read-through by a polymerase. In another embodiment, nucleic acids can include orthogonal 3' and 5' functional moieties which can react in separate click chemistry reactions. Such moieties can be for example, co-synthesized such that both moieties are present on the polynucleotide simultaneously at the time of a first click chemistry reaction. Alternatively, a first click chemistry reaction can be performed whereupon the orthogonal moiety can be added following the reaction. In one embodiment, one orthogonal group, after click chemistry yields a polynucleotide having modified backbone linkage that can be read-through by a polymerase and the second orthogonal group after click chemistry yields a polynucleotide having a modified backbone linkage than can be either non-read-through or read-through for a polymerase.

Where amplification of the ligated-nucleic acids is performed, the polymerase can be a mutated-polymerase. Such polymerases are well known in the art and include at least one amino acid mutation from the native polypeptide sequence. Mutated polymerases can have varied properties from their native counterparts including, but not limited to, greater stability, greater processivity, greater binding affinity, or any combination thereof.

In certain embodiments, the cellular nucleic acids used in the methods described herein comprise nucleic acid fragments. Fragmentation can be performed as understood by those skilled in the art. Fragmentation can also occur naturally (i.e. before extraction from the sample). In certain instances, it is advantageous to fragment the nucleic acids in the sample before performing a click chemistry reaction. In other instances, the cellular nucleic acids are fragmented at the time of collection or isolation from the sample. Cellular nucleic acids and cellular nucleic acid fragments described herein can be obtained from a sample obtained from any source (e.g. bacterial, viral, yeast, or mammal). The sample can be taken from a mammal. In some embodiments, the sample is taken from a human. The human can be a human patient (e.g. a human diagnosed with a particular disease or condition). Cellular nucleic acids and cellular nucleic acid fragments can also be obtained from various "sources" in the cell itself such as, for example, genomic DNA (gDNA), mitochondrial DNA, or plastomic DNA. In certain embodiments, the cellular nucleic acids and cellular nucleic acid fragments are obtained from gDNA. In one embodiment, the cellular nucleic acids can be obtained from human gDNA. In another embodiment, the cellular nucleic acids can be obtained from microbial gDNA.

In some embodiments, the cellular nucleic acids are attached to a solid support before the incorporation of the terminal 3'-modified ddNTP. In another embodiment, the cellular nucleic acids are attached to a solid support before the click chemistry reaction is performed. In one example, the methods described herein comprise: (a) attaching the cellular nucleic acids to a solid support before performing steps (i) and (ii) of the methods described herein for preparing a nucleic acid library.

In another embodiment, the methods described herein comprise: (a) attaching the plurality of ligated-nucleic acids to a solid support under conditions for hybridization where the solid support comprises (1) a plurality of capture primers each having a nucleic acid sequence complementary to the plurality of adaptor nucleic acids and (2) a plurality of 3'-universal primers. The methods described herein can further include extending the plurality of capture primers from step (1) above to produce a plurality of immobilized ligated-nucleic acids. The plurality of 3'-universal primers can be annealed to the immobilized ligated-nucleic acids.

In another embodiment, the methods described herein comprise (a) attaching the plurality of cellular nucleic acids to a solid support under conditions for hybridization where the solid support comprises (A) a plurality of capture primers each having a nucleic acid sequence complementary to the plurality of adaptor nucleic acids and (B) a plurality of 3'-universal primers; (b) extending the plurality of capture primers from step (A) above to produce a plurality of immobilized cellular nucleic acids; (c) incorporating a terminal 3'-modified ddNTP into the cellular nucleic acids thereby forming a plurality of 3'-modified cellular nucleic acids; and (d) reacting the 3'-modified cellular nucleic acids with a plurality of adaptor nucleic acids comprising a 5'-modified ddNTP as described herein, forming a plurality of immobilized ligated-nucleic acids. In one embodiment, the immobilized ligated-nucleic acids are sequenced according to techniques known in the, such as for example, SBS.

In one embodiment, the cellular nucleic acids of the methods described herein comprise a terminal 3'-modified ddNTP that undergoes a click chemistry reaction with a moiety attached to a solid support. In such embodiments, the cellular nucleic acids immobilized on a solid support can undergo further modification using the methods described herein.

In another aspect, the methods described herein are applicable to nanopore sequencing such as that described in U.S. Patent Publication No. 2015-0344945 and U.S. Patent Publication No. 2015-015245. In one embodiment, provided herein are methods of attaching a tether as described herein to a barrier comprising one or more nanopores. A "barrier" is intended to mean a structure that normally inhibits passage of molecules from one side of the barrier to the other side of the barrier. The molecules for which passage is inhibited can include, for example, ions or water soluble molecules such as nucleic acids, proteins, nucleotides, and amino acids. A pore (e.g. a nanopore or plurality of nanopores as described herein) can be disposed within a barrier, and the aperture of the pore can permit passage of molecules from one side of the barrier to the other side of the barrier. Barriers include membranes of biological origin, and non-biological barriers such as solid state membranes.

The tether can optionally comprise a polynucleotide sequence. The methods can include extending a tether by contacting the tether with a template independent polymerase as described herein, whereupon the template independent polymerase incorporates a terminal 3'-modified ddNTP comprising a 3'-functional moiety capable of participating in a click chemistry reaction into the tether. The 3'-functional moiety can be reacted with a 5'-functional moiety located on the barrier comprising the one or more nanopores. In certain embodiments, the 5'-functional moiety is attached to a moiety on the nanopore or the barrier adjacent to a nanopore. The moiety can be a molecule forming the barrier itself, e.g. a lipid or cholesterol in the instance of biological nanopores, or the solid support/polymer in the instance of non-biological nanopores. In one embodiment, the 3'-functional moiety can be reacted with a cholesterol-containing tag for use in translocating a polynucleotide through a nanopore as described herein and provided in, for example, U.S. Patent Publication No. 2015/015245, International Patent Publication No. WO2015/081211, and International Patent Application No. PCT/US2014/067560.

In another aspect, the methods described herein can be applicable to nanopore sequencing by anchoring or attaching a plurality of cellular nucleic acids to a barrier comprising a plurality of nanopores. In one embodiment, a sample comprising the plurality of cellular nucleic acids each having a terminal 3'-modified ddNTP comprising a 3'-functional moiety capable of participating in a click chemistry reaction, is reacted with a barrier comprising a plurality of nanopores, wherein the barrier or the nanopore comprises a compatible 5'-functional moiety capable of participating in a click chemistry reaction with the 3' functional moiety, wherein the 3' functional moiety and 5' functional moiety react to form a modified backbone linkage, thereby forming a plurality of ligated-nucleic acids. In one embodiment, the barrier or nanopore comprises a terminal 5'-modified ddNTP as described herein. In one embodiment, the plurality of ligated-nucleic acids is amplified thereby preparing a nucleic acid library from the sample.

As used herein, the term "pore" is intended to mean a structure that includes an aperture that permits molecules to cross from a first side of the pore to a second side of the pore. That is, the aperture extends through the first and second sides of the pore. Molecules that can cross through an aperture of a pore can include, for example, ions or water-soluble molecules such as nucleic acids, proteins, nucleotides, and amino acids. The pore can be disposed within a barrier. When at least a portion of the aperture of a pore has a width of 100 nm or less, e.g., 10 nm or less, or 2 nm or less, the pore can be, but need not necessarily be, referred to as a "nanopore." Optionally, a portion of the aperture can be narrower than one or both of the first and second sides of the pore, in which case that portion of the aperture can be referred to as a "constriction." Alternatively or additionally, the aperture of a pore, or the constriction of a pore (if present), or both, can be greater than 0.1 nm, 0.5 nm, 1 nm, 10 nm or more. A pore can include multiple constrictions, e.g., at least two, or three, or four, or five, or more than five constrictions.

Nanopores useful in the invention described herein include first and second sides and an aperture that extends through the first and second sides. In one embodiment, the 3'-functional moiety reacts with a to the first side (e.g. "outside" of the nanopore). In another embodiment, the 3'-functional moiety attaches to the second side (e.g. "inside" of the nanopore). The tether can be a permanent tether as provided in U.S. Patent Publication No. 2015-0344945. As used herein, "tether" is intended to mean an elongated member having a head region, a tail region, and an elongated body therebetween. A tether can include a molecule. A tether can be, but need not necessarily be, in an elongated state, e.g., can include an elongated molecule. For example, an elongated body of a tether can have secondary or tertiary configurations such as hairpins, folds, helical configurations, or the like. Tethers can include polymers such as polynucleotides or synthetic polymers. Tethers can have lengths (e.g., measured in a stretched or maximally extended state) ranging, for example, from about 5 nm to about 500 nm, e.g., from about 10 nm to about 100 nm. Tethers can have widths ranging, for example, from about 1 nm to about 50 nm, e.g., from about 2 nm to about 20 nm. Tethers can be linear or branched. A tether can be considered to be "permanent" when it is not removed during a detection method.

As used herein, a "head region" of a tether is intended to mean a functional group of the tether that is attached to another member. In one embodiment, the head region comprises a 3'-functional moiety capable of participating in a click chemistry reaction as described herein. In one embodiment, such attachment can be formed through hybridization of a terminal 3'-modified ddNTP described herein comprising the head region to a molecule comprising terminal 5'-modified ddNTP comprising a compatible 5'-functional moiety capable of participating in a click chemistry reaction with the 3' functional moiety.

As used herein, a "tail region" of a tether is intended to mean a portion of the tether that is disposed distally from the head region. The tail region can extend freely away from the head region, e.g., can be unattached to any other member. In one embodiment, the tail region comprises a 3'-functional moiety capable of participating in a click chemistry reaction as described herein. In one embodiment, such attachment can be formed through hybridization of a terminal 3'-modified ddNTP described herein comprising the tail region to a molecule comprising terminal 5'-modified ddNTP comprising a compatible 5'-functional moiety capable of participating in a click chemistry reaction with the 3' functional moiety.

As used herein, an "elongated body" is intended to mean a portion of a member, such as a tether, that is sufficiently long and narrow to be disposed within at least a portion of an aperture of a pore. An elongated body can be formed of any suitable material of biological origin or nonbiological origin, or a combination thereof. In one example, the elongated body includes a polymer as described herein.

In one embodiment, the terminal 3'-modified ddNTP is a component of a permanent tether as provided in U.S. Patent Publication No. 2015-0344945. In one embodiment, the tether containing the terminal 3'-modified ddNTP attaches to one of the sides of the nanopore via a head region. In one embodiment, the tether containing the terminal 3'-modified ddNTP attaches to one of the sides of the nanopore via a tail region. Tethers useful in the inventions described herein can include an elongated body disposed therebetween as provided in U.S. Patent Publication No. 2015-0344945. The tether can include one or more features (e.g. reporter region(s)) that facilitates detection of an event. In certain embodiments, the detection of the event is detection of one or more nucleic acids and can facilitate sequencing of a polynucleotide. In one embodiment, the event occurs as a result of application of current or flux. In certain embodiments, the detection of the event is dependent upon the movement of the reporter molecule within the nanopore as described in 12957-178-999.

In some embodiments, the tether is anchored to a protein. In one embodiment, the event occurs as a result of a first conformational change of the protein that can move the head region, and the movement of the head region can translationally move the reporter region. In some embodiments, the protein includes an enzyme. For example, the enzyme can include a polymerase. The first conformational change can occur responsive to the polymerase acting upon a first nucleotide. In some embodiments, the terminal 3'-modified ddNTP attaches the protein to a nanopore.

The methods described herein can be used in single molecule sequencing methods. For example, in one embodiment, a plurality of cellular nucleic acids each having a terminal 3'-modified dideoxynucleotide (ddNTP) comprising a 3'-functional moiety capable of participating in a click chemistry reaction can be attached to a barrier comprising a plurality of nanopores to hold or guide the cellular nucleic acids during sequencing by nanopore sequencing.

In other embodiments, the methods include reacting a sample comprising a plurality of cellular nucleic acids each having a terminal 3'-modified dideoxynucleotide (ddNTP) comprising a 3'-functional moiety capable of participating in a click chemistry reaction, with a plurality of polymers each comprising a 5'-functional moiety capable of participating in a click chemistry reaction with the 3' functional moiety, wherein the 3' functional moiety and 5' functional moiety react to form a modified backbone linkage, thereby forming a plurality of ligated-nucleic acids. In one embodiment, the ligated-nucleic acids are amplified as described herein. In one embodiment, the polymer is a molecule forming a barrier comprising a plurality of nanopores as described herein.

Polymers useful in the inventions described herein can be biological or synthetic polymers. Exemplary biological polymers include polynucleotides, polypeptides, polysaccharides, polynucleotide analogs, and polypeptide analogs. Exemplary polynucleotides and polynucleotide analogs include DNA, enantiomeric DNA, RNA, PNA (peptide-nucleic acid), morpholinos, and LNA (locked nucleic acid). Exemplary synthetic polypeptides can include charged amino acids as well as hydrophilic and neutral residues. Exemplary synthetic polymers include PEG (polyethylene glycol), PPG (polypropylene glycol), PVA (polyvinyl alcohol), PE (polyethylene), LDPE (low density polyethylene), HDPE (high density polyethylene), polypropylene, PVC (polyvinyl chloride), PS (polystyrene), NYLON (aliphatic polyamides), TEFLON^{®} (tetrafluoroethylene), thermoplastic polyurethanes, polyaldehydes, polyolefins, poly(ethylene oxides), poly(ω-alkenoic acid esters), poly(alkyl methacrylates), and other polymeric chemical and biological linkers such as described in Hermanson, Bioconjugate Techniques, third edition, Academic Press, London (2013).

In some embodiments, the adaptor nucleic acids are attached to a solid support before the reacting. In certain embodiments, the solid support is a nanopore or bead (e.g. polymeric bead). In another embodiment, the adaptor nucleic acids are attached to a solid support before the terminal 5'-modified dideoxynucleotide (ddNTP) is incorporated into the cellular nucleic acids.

In one embodiment, the plurality of cellular nucleic acids is amplified before incorporating a terminal 3'-modified ddNTP. Amplification of the plurality of cellular nucleic acids before incorporating a terminal 3'-modified ddNTP can be done using techniques known and understood in the art. In one embodiment, the plurality of cellular nucleic acids is not amplified before incorporating a terminal 3'-modified ddNTP.

The library of ligated-nucleic acids can be sequenced using techniques known in the art. For example, sequencing can be performed using Sequencing by Synthesis (SBS) as known in the art.

In one embodiment, the methods described herein further comprise contacting the plurality of ligated-nucleic acids to a solid support under conditions for hybridization (e.g. incubation for 5, 10, 15, 20, 30, 60, 90, 120 minutes or less), wherein the solid support comprises (1) a plurality of capture primers each having a nucleic acid sequence complementary to the plurality of adaptor nucleic acids and (2) a plurality of 3'-universal primers. In one embodiment, after capture of the adaptor nucleic acids, the plurality of capture primers is extended to produce a plurality of immobilized ligated-nucleic acids. In still another embodiment, the methods described herein further comprise annealing the plurality of universal primers to the immobilized ligated-nucleic acids.

In another embodiment, the methods described herein further comprise contacting the plurality of ligated-nucleic acids to a solid support comprising a plurality of 3'-universal primers. In one embodiment, after capture of the adaptor nucleic acids, the plurality of capture primers is extended to produce a plurality of immobilized ligated-nucleic acids. In still another embodiment, the methods described herein further comprise annealing the plurality of universal primers to the immobilized ligated-nucleic acids.

In another embodiment, the method of preparing a nucleic acid library from a sample comprises: (i) reacting a sample comprising a plurality of cellular nucleic acids each having a terminal 3'-modified dideoxynucleotide (ddNTP) comprising a 3'-functional moiety capable of participating in a click chemistry reaction, wherein the 3'-modified ddNTP, with a plurality of molecular tethers, each of the plurality of molecular tethers comprising a functional moiety capable of participating in a click chemistry reaction with the 3' functional moiety, wherein the 3' functional moiety and functional moiety of the molecular tether, thereby forming a plurality of ligated-cellular nucleic acids; and (ii) amplifying the plurality of ligated-cellular nucleic acids thereby preparing a nucleic acid library from the sample..

In certain embodiments, the sample comprises a plurality of cellular nucleic acids comprising 20 ng, 15 ng, 10 ng, 5 ng, 1 ng, or less input nucleic acid.

The methods of preparing a nucleic acid library described herein can be completed in a start-to-finish time comprising 10, 9, 8, 7, 6, 5, 4, 3, 2, 1, or less hours.

Also provided herein is a method of preparing a nucleic acid library from a sample , the method comprising; reacting a sample comprising a plurality of cellular nucleic acids each comprising a terminal 3'-modified dideoxynucleotide (ddNTP) comprising a 3'-functional moiety capable of participating in a chemical ligation reaction, wherein the 3'-modified ddNTP is incorporated into each of the plurality of cellular nucleic acids by contacting the plurality of cellular nucleic acids with a template independent polymerase, with a plurality of adaptor nucleic acids each comprising a terminal 5'-modified ddNTP comprising compatible 5'-functional moiety capable of participating in a chemical ligation reaction with the 3' functional moiety, wherein the 3' functional moiety and 5' functional moiety react to form a modified backbone linkage, thereby forming a plurality of ligated-nucleic acids; and amplifying the plurality of ligated-nucleic acids thereby preparing a nucleic acid library from the sample.

In still another embodiment of the methods of preparing a nucleic acid library from a sample as claimed in the appended claims, is a method comprising:
(i) reacting a sample comprising a plurality of cellular nucleic acids each comprising a terminal 3'-modified dideoxynucleotide (ddNTP) comprising a 3'-functional moiety capable of participating in a click chemistry reaction, wherein the terminal 3'-modified ddNTP is incorporated into each of the plurality of cellular nucleic acids by contacting the plurality of cellular nucleic acids with a template independent polymerase, with a plurality of adaptor nucleic acids each comprising a terminal 5'-modified ddNTP comprising compatible 5'-functional moiety capable of participating in a click chemistry reaction with the 3' functional moiety, wherein the 3' functional moiety and 5' functional moiety react to form a modified backbone linkage, thereby forming a plurality of ligated-nucleic acids;
(ii) contacting the plurality of ligated-nucleic acids to a solid support under conditions for hybridization, wherein the solid support comprises (1) a plurality of capture primers having a nucleic acid sequence complementary to the plurality of adaptor nucleic acids, and (2) a plurality of universal primers;
(iii) extending the plurality of capture primers to produce a plurality of immobilized target nucleic acids complementary to the ligated-nucleic acids;
(iv) annealing the plurality of universal primers to the immobilized target nucleic acids; and
(v) amplifying by polymerase chain reaction (PCR) the plurality of immobilized target nucleic acids.

In another aspect of the methods described herein is a method of capturing DNA obtained from a limited number of cells for DNA library preparation. In one embodiment, the method comprises reacting a plurality of cellular DNA fragments comprising a terminal 3'-modified ddNTP as described herein comprising a 3'-functional moiety capable of participating in a click chemistry reaction as described herein, wherein the terminal 3'-modified ddNTP is incorporated into each of the plurality of cellular nucleic acids by contacting the plurality of cellular nucleic acids with a template independent polymerase provided herein, wherein the plurality of cellular DNA fragments is obtained from a sample obtained from a limited number of cells, with a plurality of adaptor nucleic acids each comprising a terminal 5'-modified ddNTP comprising compatible 5'-functional moiety capable of participating in a click chemistry reaction with the 3' functional moiety as provided herein, wherein the 3' functional moiety and 5' functional moiety react to form a modified backbone linkage; and contacting the plurality of ligated-nucleic acids to a solid support as described herein under conditions for hybridization, wherein the solid support comprises (1) a plurality of capture primers having a nucleic acid sequence complementary to the plurality of adaptor nucleic acids, thereby capturing DNA obtained from a single cell.

In another embodiment, the method comprises reacting a sample comprising a plurality of cellular DNA fragments comprising a terminal 3'-modified ddNTP as described herein comprising a 3'-functional moiety as provided herein capable of participating in a click chemistry reaction obtained from a single cell, wherein the terminal 3'-modified ddNTP is incorporated into each of the plurality of cellular nucleic acids by contacting the plurality of cellular nucleic acids with a template independent polymerase as provided above, with a plurality of adaptor nucleic acids as described herein attached to a solid support as described herein, wherein each of the plurality of adaptor nucleic acids comprise a terminal 5'-modified ddNTP comprising compatible 5'-functional moiety capable of participating in a click chemistry reaction as described herein, with the 3' functional moiety, wherein the 3' functional moiety and 5' functional moiety react to form a modified backbone linkage.

In another aspect of the methods described herein is a method of selectively cleaving a single strand of a double stranded polynucleotide sequence. In one embodiment, the method comprises: (i) preparing a template strand by reacting a first polynucleotide comprising a terminal 3'-modified ddNTP comprising a 3'-functional moiety capable of participating in a click chemistry reaction, wherein the terminal 3'-modified ddNTP is incorporated into the first polynucleotide by contacting the first polynucleotide with a template independent polymerase, with a second polynucleotide comprising a terminal 5'-modified ddNTP comprising compatible 5'-functional moiety capable of participating in a click chemistry reaction with the 3' functional moiety, wherein the 3' functional moiety and 5' functional moiety react to form a modified backbone linkage, wherein the template strand comprises a first restriction site that comprises the modified backbone linkage; (ii) extending the first or second polynucleotide to produce a double stranded nucleic acid, wherein the complementary strand of the double stranded nucleic acid comprises a second restriction site complementary to the first restriction site; (iii) contacting the double stranded nucleic acid with a nucleic acid-cleaving enzyme; and (iv) cleaving the double stranded nucleic acid with the nucleic acid-cleaving enzyme, wherein the nucleic acid-cleaving enzyme recognizes the first and second restriction sites and cleaves only at the second restriction site, forming a 5'-primer sequence and a 3'-strand.

In one embodiment, the first polynucleotide is part of a plurality of polynucleotides.

In one embodiment, the double stranded nucleic acid is produced by extending from the second polynucleotide.

In one embodiment, the DNA cleaving enzyme is a restriction endonuclease (REase) or a nicking endonuclease (NEase). In another embodiment, the DNA cleaving enzyme is a restriction endonuclease (REase). REases are well known in the art. REases useful in the methods described herein include REases capable of recognizing the desired recognition sequences to cleave polynucleotides described herein. For example, the REase can be a Type I, Type II, Type III or Type IV REase. REases useful in the methods described herein can clean inside of their recognition sequence or outside their recognition sequence as is known in the art. NEases useful in the methods described herein include NEases capable of recognizing the desired recognition sequences to cleave polynucleotides described herein. NEases can hydrolyze a single strand of a double stranded polynucleotide within or outside of the recognition sequence of the NEase.

In a particular embodiment of the methods to selectively cleave a single strand of a double stranded polynucleotide sequence described herein, the template independent polymerase is TdT.

In one embodiment, of the methods to selectively cleave a single strand of a double stranded polynucleotide sequence described herein, the method further comprises (v) extending from the 5'-primer sequence, thereby displacing the 3'-strand.

In another embodiment, steps (iii) to (v) of the method of selectively cleaving a single strand of a double stranded polynucleotide sequence are repeated iteratively over at least 5, 10, 15, 20, 25, 50, or more cycles.

It is understood that modifications which do not substantially affect the activity of the various embodiments of this invention are also included within the definition of the disclosure provided herein. Accordingly, the following examples are intended to illustrate but not limit the present invention.

### Examples:

Installation of azido- or alkynyl- modifications. The workflow for the methods described herein is illustrated in Scheme 1. Step 1 involves incorporation of a 3'-azido modified ddNTP. Upon successful incorporation, the DNA library will be modified with a 3' azido group. This can then be chemical ligated to a 5'-alkynyl modified adapter in a copper-assisted click reaction.

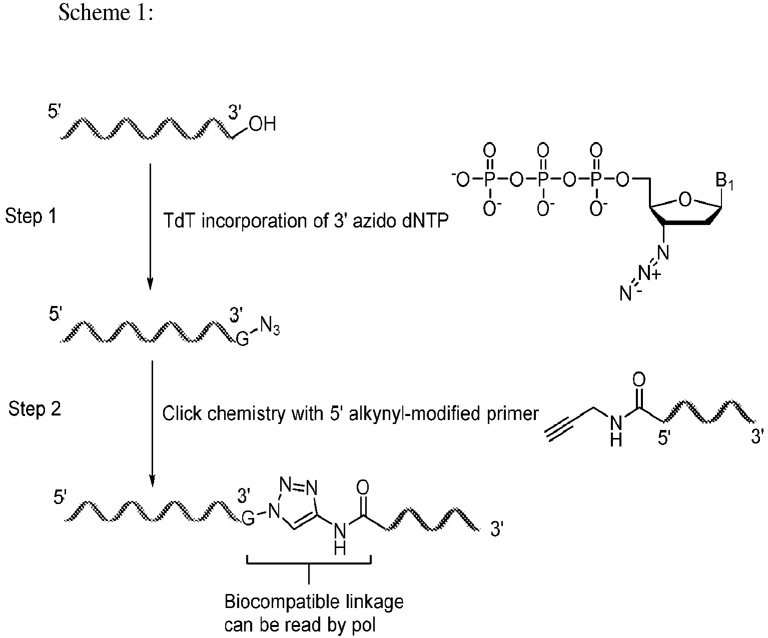

The 3'-azido nucleotide triphosphates are available commercially. The 3'-alkynyl nucleotide triphosphates are not readily available commercially. Here, we report the synthesis of these novel nucleotides as illustrated in Schemes 2 and 3.

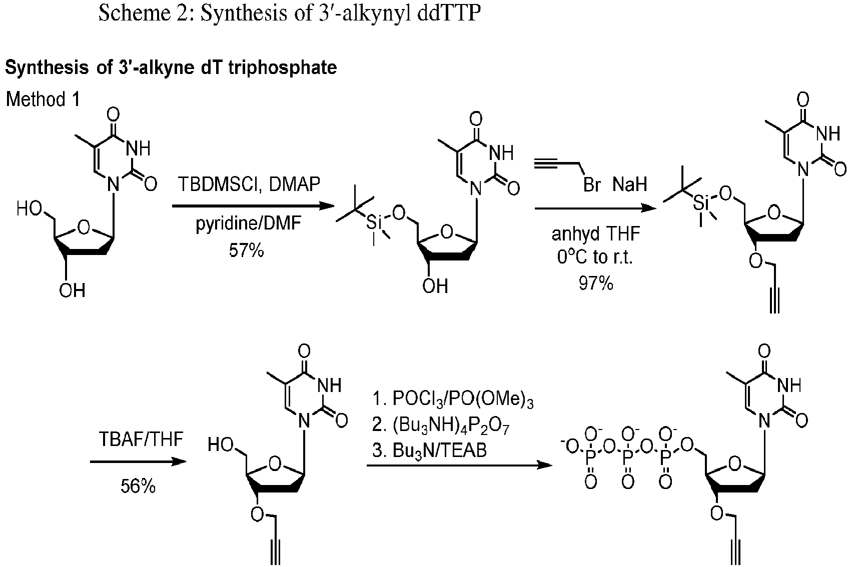

Starting from thymidine, the 5-hydroxyl was protected using TBDMSCl in pyr/DMF, providing a 57% yield. The alkynyl group was installed using propargyl bromide and NaH in THF providing 97% yield of the alkynyl intermediate. Following deprotection of hydroxyl and installation of the triphosphate the final product was obtained in a total % yield of 3-6%.

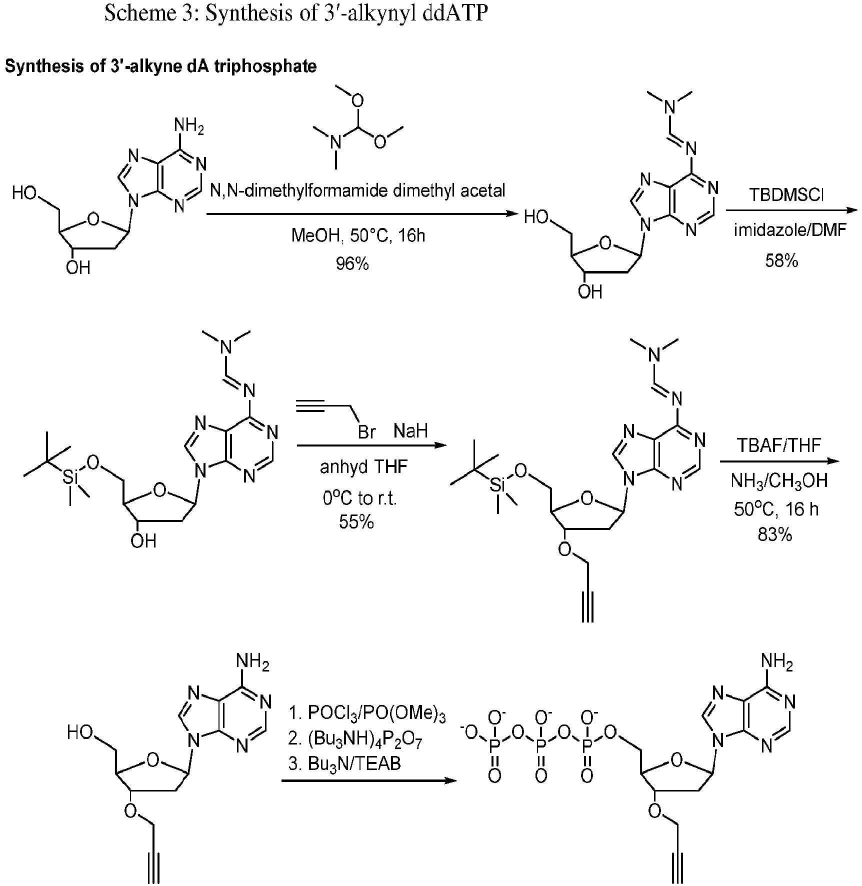

Synthesis of ddATP was performed in a manner consistent with Scheme 2.

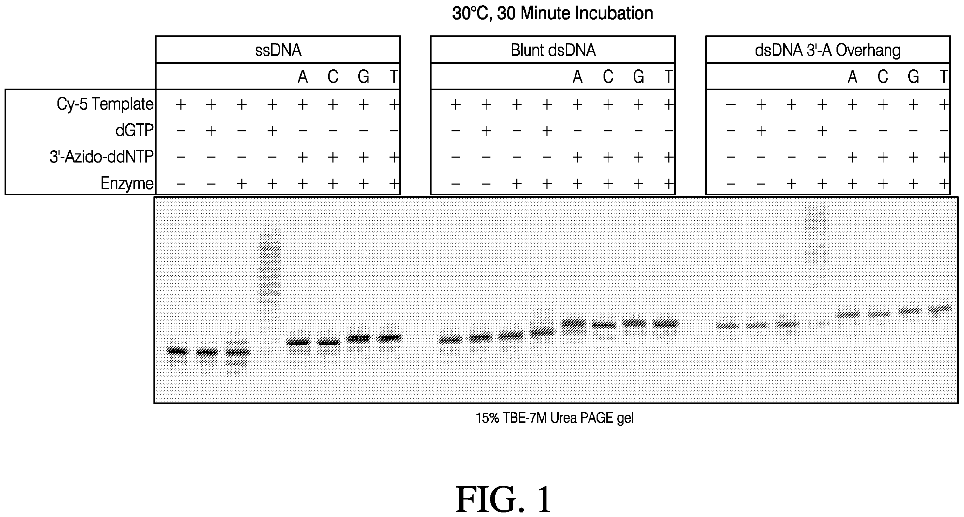

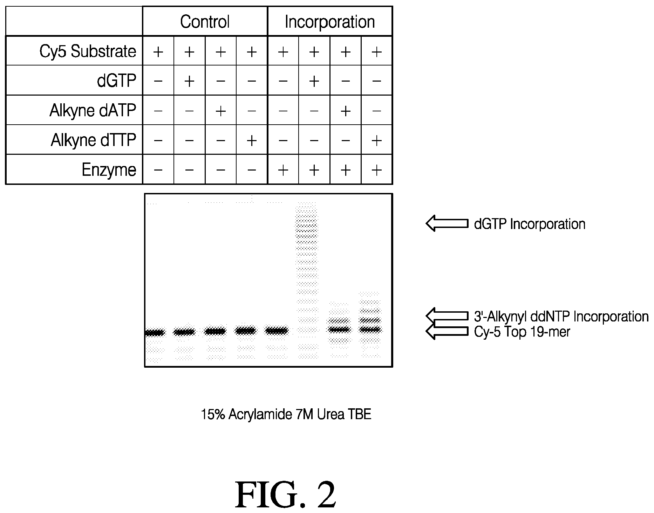

TdT incorporation of 3' azido nucleotides and 3' alkynyl nucleotides. TdT could incorporate 3' azido ddNTPs on single stranded DNA and dsDNA with both blunt ends and with A-overhang as shown in FIG. 1. In addition, the 3' alkynyl nucleotide triphosphates synthesized de novo herein, could also be incorporated by TdT as shown in FIG. 2. The efficiencies of the incorporation of 3' alkynyl nucleotides appeared less efficient than that of the 3' azido nucleotides. Without being bound by any particular theory, this could be likely due to the larger size of the group at the 3'-hydroxyl. The binding pocket of TdT enzyme near that 3' OH is tight fitting, and smaller groups may be better accommodated.

Successive TdT and click reaction for click ligation of DNA templates. Since the TdT incorporation yields of the 3' azido nucleotides are higher and almost quantitative, experiments were performed using the 3' azido/5' alkyne format.

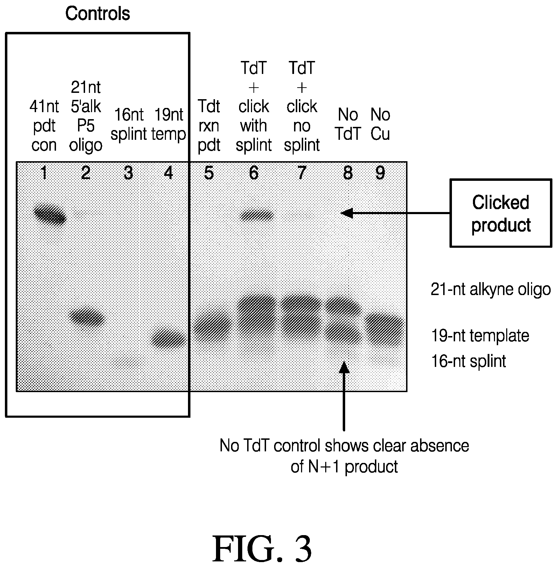

Upon successful incorporation of the 3' azido ddNTP, the next step is to perform chemical ligation with a 5'-alkynyl modified oligonucleotide in a click ligation step. This step was performed successfully as seen in FIG. 3. In Lane 6 of FIG. 2, the 3'-azido product from the TdT reaction was reacted directly with a 5'-alkynyl DNA oligonucleotide, in the presence of the Cu(I) catalyst generated *in situ* from copper (II) sulfate, sodium ascorbate and tris(3-hydroxypropyltriazolylmethyl)amine (THPTA) ligand. In control experiments where TdT enzyme is absent (Lane 8), no ligation product was observed. In addition, the TdT (n+1) product was visibly absent as well. When the copper catalyst was absent (Lane 9), no ligation product was observed. This indicates that the ligation product was indeed formed from Cu-assisted click ligation.

Table of Conditions for ligation shown in FIG. 3.

| Reagent | Initial Conc | Final conc | Vol | No of moles |
|---|---|---|---|---|
| TdT Buffer | 10X | 1X | 2 uL | |
| 2.5 mM CoCl₂ | 10X | 1X | 2 uL | |
| 19nt-Template | 25 uM | 2.5 uM | 2 uL | 50 picomoles |
| Nucleotide | 10 mM | 2.5 mM | 5 uL | 50 nanomoles |
| Enzyme | 20 units/uL | 2 units/uL | 5 uL | 100 units |
| Water | | | 4 uL | |
| | | Total volume | 20 uL | |

Reported conc:
TdT rxn: 0.2 µM
Click rxn: 200 µM
Nucleotide = 3' N₃-ddT
30 min TdT incubation
Heat inactivation for 20 min at 75 °C
Click reaction at 0 °C for 1 h and room temperature of 1 h.

This ligation represents the first demonstration of a successive TdT and click ligation performed on natural DNA templates. Unlike previous reports of click ligation on DNA which were performed using chemically synthesized DNA with azido or alkynyl modifications, this click ligation was performed on natural DNA templates that are modified enzymatically using TdT enzyme and can be applied to a natural DNA library.

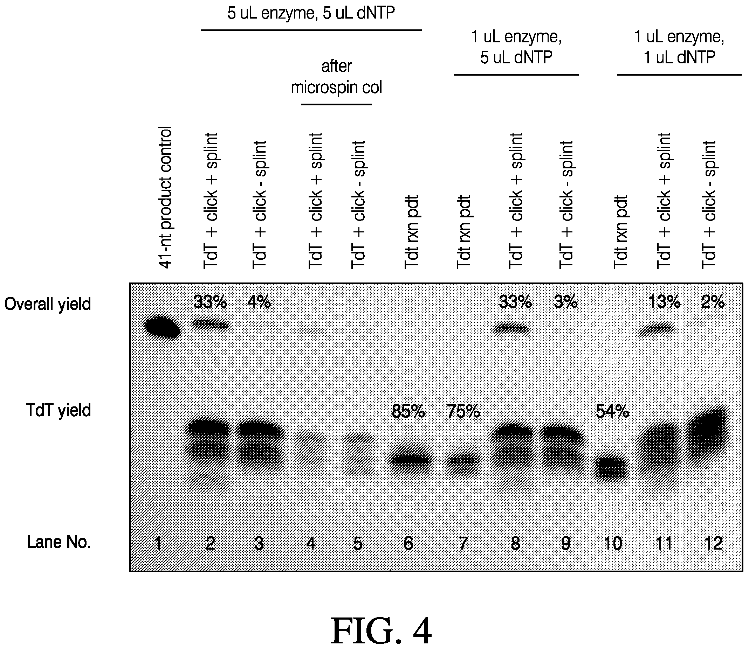

Conditions for TdT-assisted click ligation for DNA oligonucleotides were investigated. The concentration of TdT enzyme and 3'-azido dNTP nucleotide was varied in FIG. 4. A low nucleotide concentration resulted in significantly less product formed. The TdT enzyme concentration could be decreased by 5-fold without significant loss in reaction efficiency.

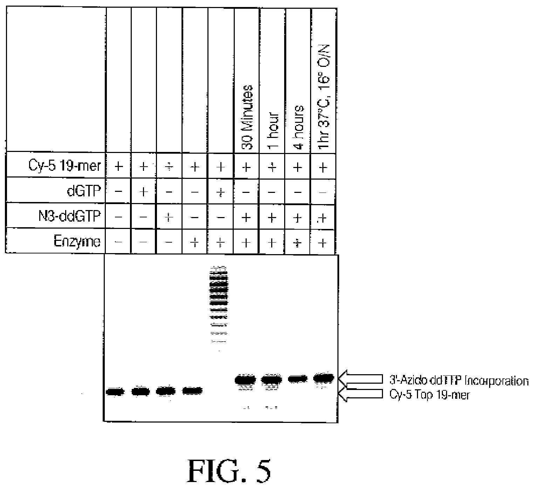

Experiments to vary the length of TdT reaction were completed (FIG. 5). No significant increase in yield was observed with longer TdT incubation time. The reaction was efficiently carried out within 1h for the incorporation of 3'-azido ddTTP. However, TdT efficiently incorporated the 3-azido ddNTP within 30 min of incubation time.

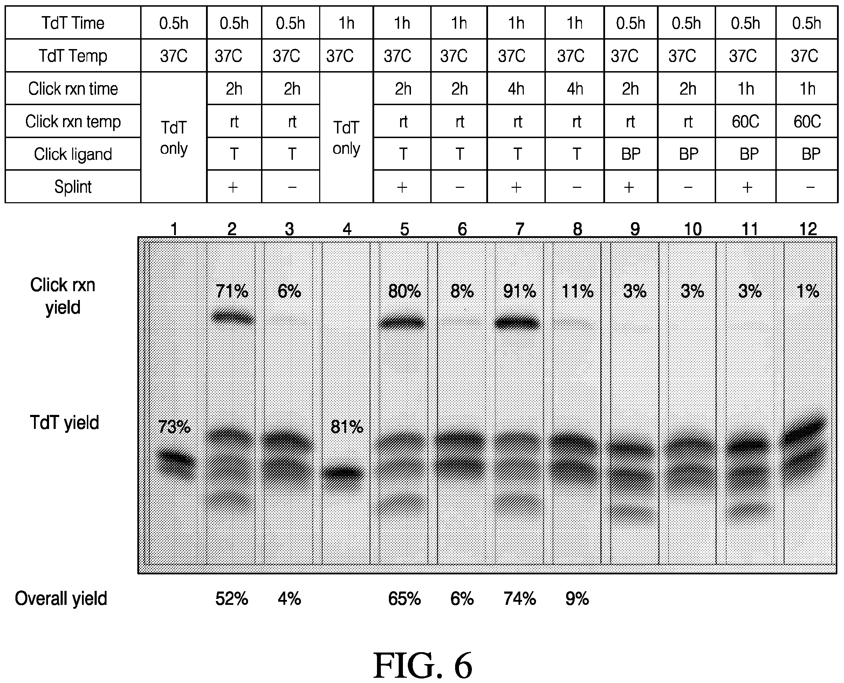

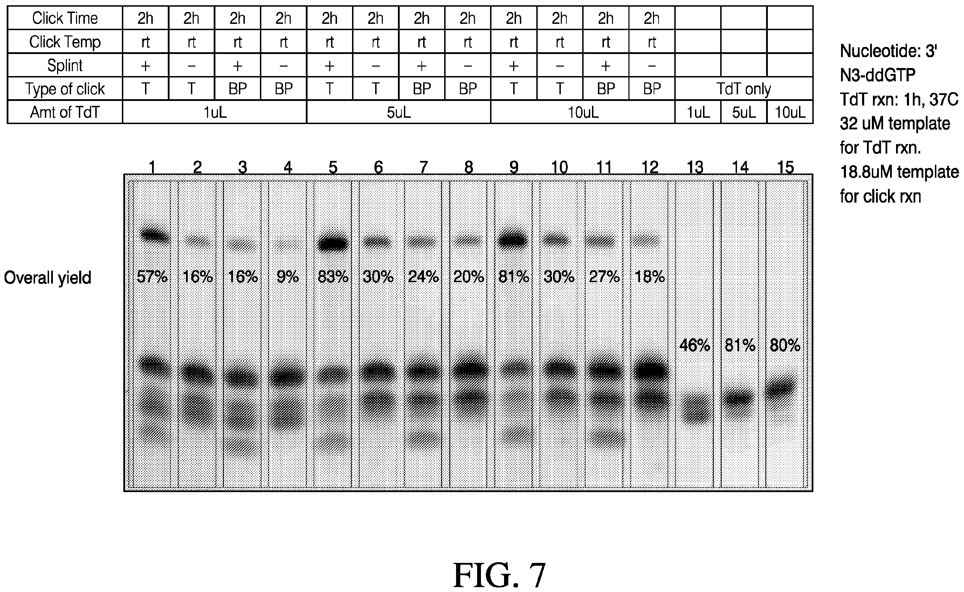

Consecutive TdT and click ligation was performed on different amounts of template as shown in FIG. 6 and FIG. 7. In FIG. 6, 1.55 µM of TdT product and 5' alkynyl-DNA oligonucleotides were used in the click reaction. The TdT reaction yields were 73% to 81%. The click reaction yield increased from 71% to 91% when the length of click reaction was increased from 2h to 4h. An overall yield of 74% was achieved when the TdT reaction was carried out for 1h and the click reaction was performed for 4h. Black pool conditions resulted in a 3% click ligation yield.

The yields were further improved when the click reaction was carried out at a higher concentration. When the concentration of TdT product and 5' alkynyl-oligo was increased to 18.8 µM, the overall TdT and click reaction yield was above 80% (FIG. 7). This reaction was carried out in the presence of a splint oligo. Without a splint, the reaction yield was 30% under identical conditions. The highest obtained yield was completed using THPTA ligand during the click chemistry reaction.

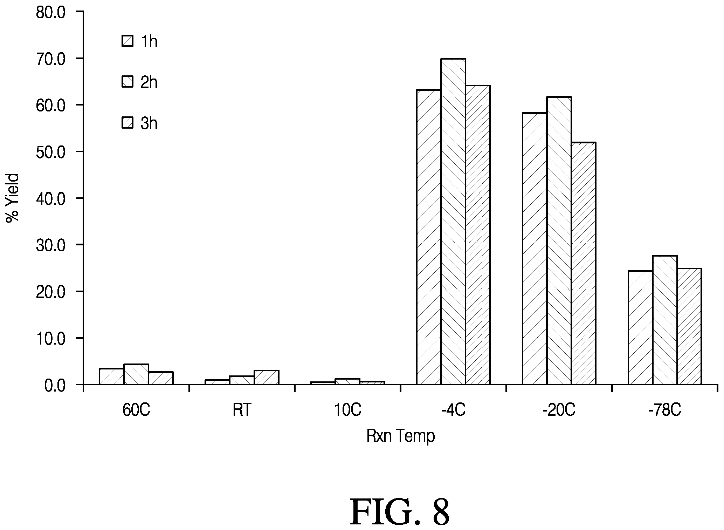

The yield of the click reaction without a splint was further increased by using conditions discovered *inter alia.* Click reactions performed below freezing temperatures resulted in much higher yields. Without being bound by any particular theory, this may be likely due to the increase in the effective concentration of the DNA template in solution when water gradually crystallized as the reaction was incubated at -4°C or -20°C (FIG. 8). The concentration of DNA used in the experiment was 0.9 µM.

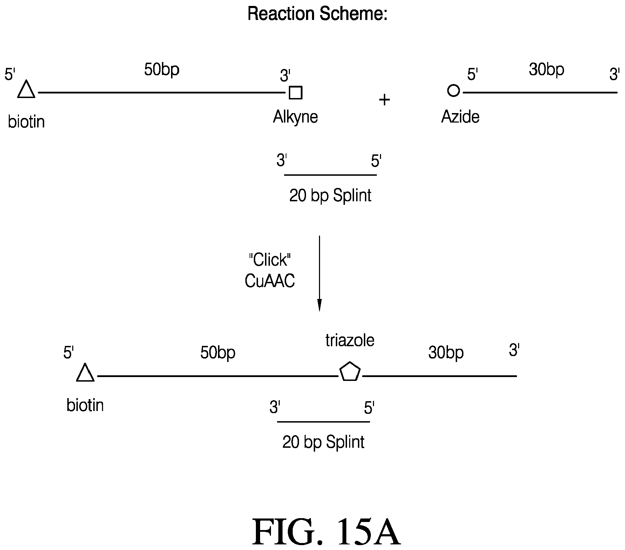

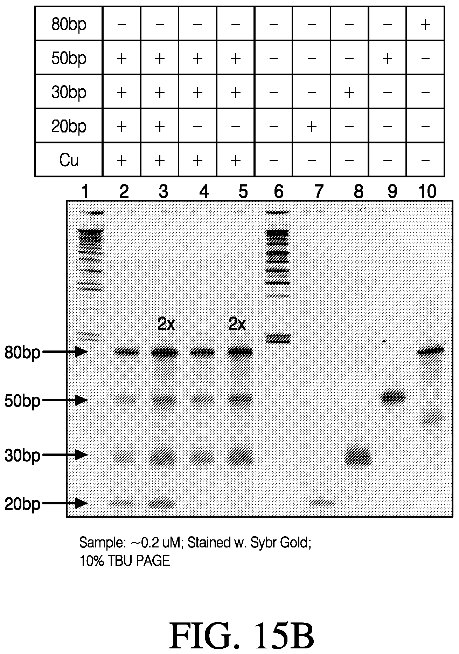

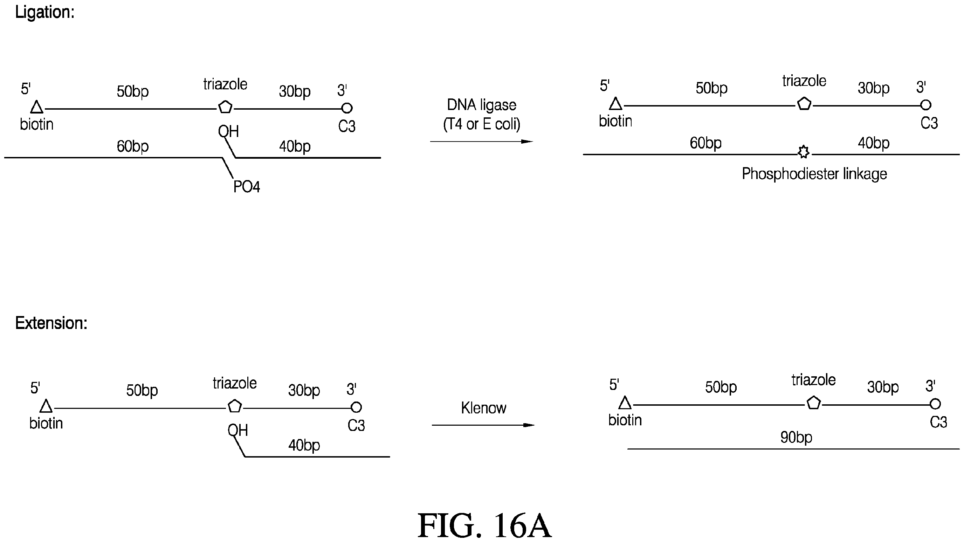

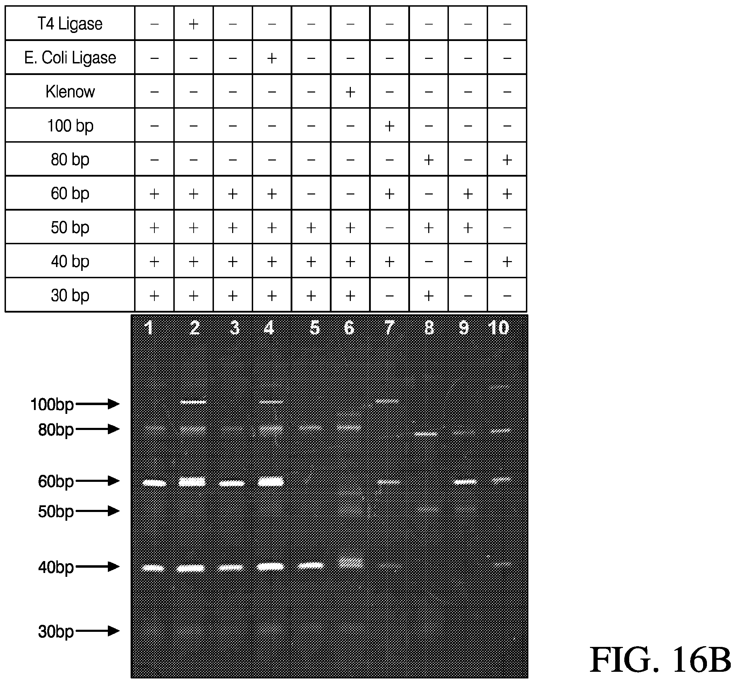

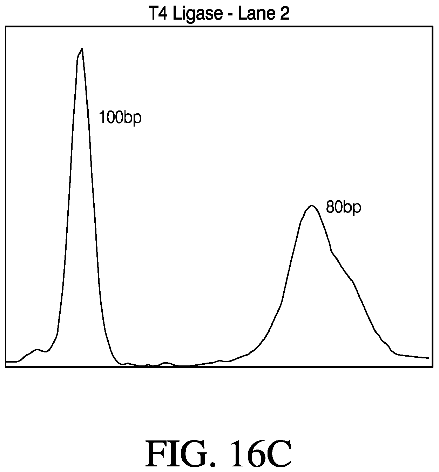

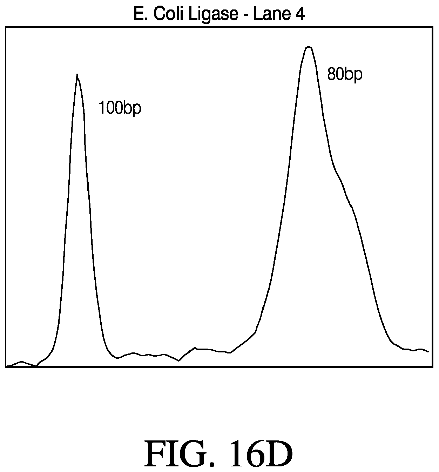

Reactions were also performed using a 50 bp natural DNA sequence having a 3' alkynyl functional group and a 30 bp adaptor sequence having an azido functional group. As provided in FIG. 15B, the reaction proceeded smoothly to create the ligated 80 bp product. Similar experiments were demonstrated in FIGS. 16A-16B showing successful ligation by T4 ligase of a 60 bp and 40 bp complement to a ligated-triazolyl containing template strand.

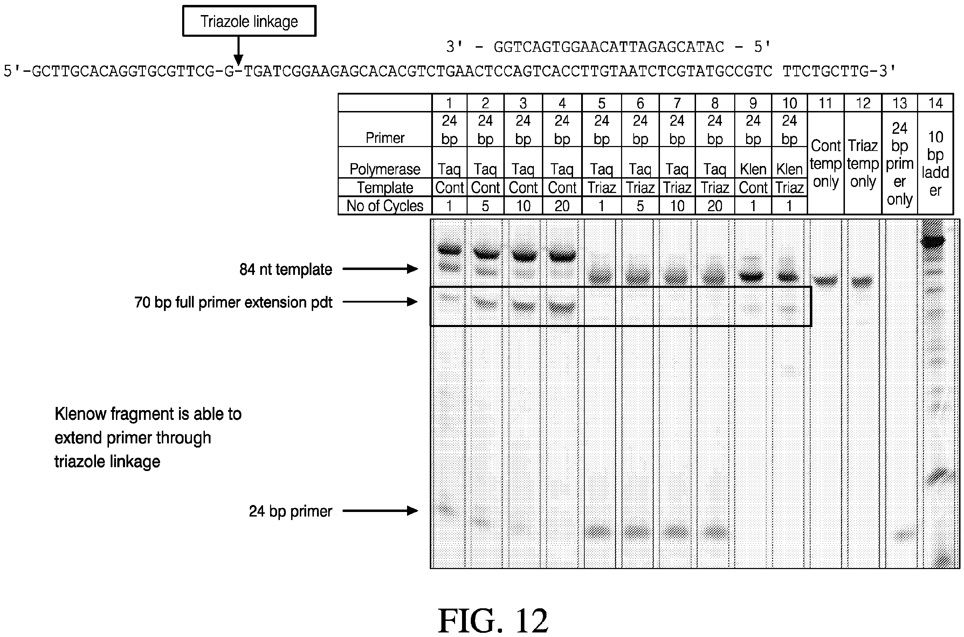

Experiments were performed to demonstrate conclusively that the Klenow fragment could extend a 24 bp primer (SEQ ID NO: 1, 3'-GGTCAGTGGAACATTAGAGCATAC-5') through the triazole linker of the ligated sequence (SEQ ID NO: 2, 5'-GCTTG CACAG GTGCG TTCG-G-(triazole linkage)-TGATC GGAAG AGCAC ACGTC TGAAC TCCAG TCACC TTGTA ATCTC GTATG CCGTC TTCTG CTTG-3'(FIG. 12).

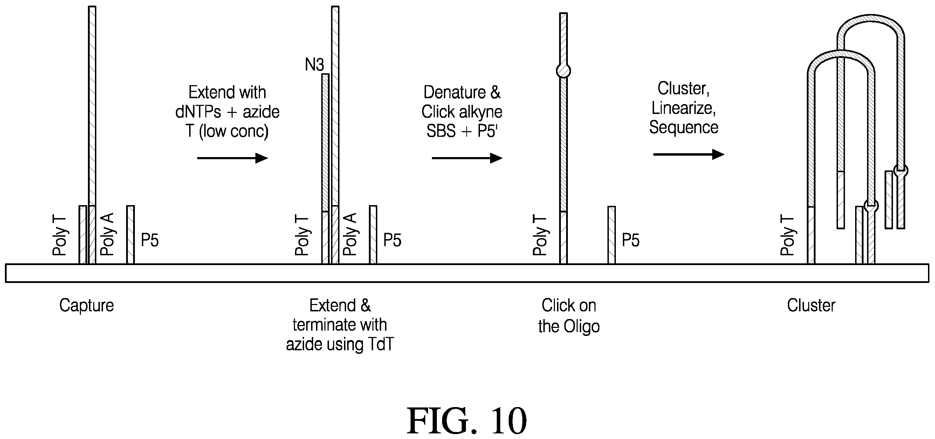

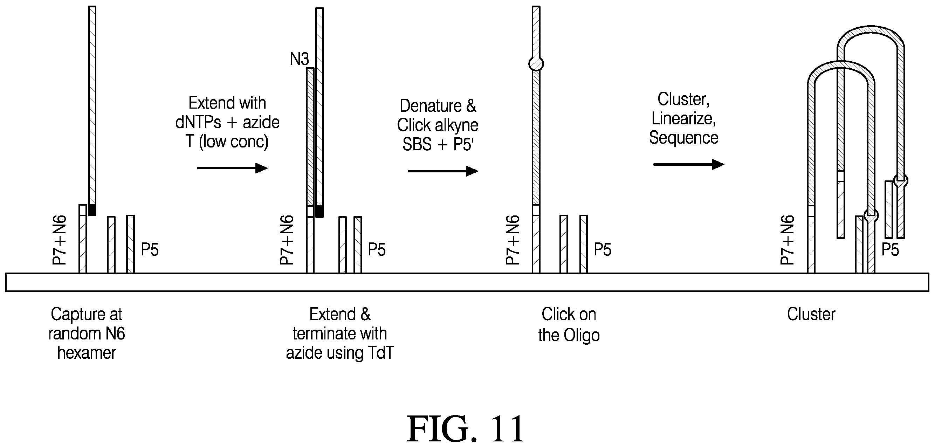

Clustering Reactions of Chemically Ligated Library Nucleic Acids. The methods described herein are applicable to library preparation and surface capture methods using a TdT assisted modification of DNA library. An example is the direct capture of RNA library on the flow cell surface as provided in FIG. 10. The click reaction eliminates the enzymatic reaction to attach the necessary adaptors for performing SBS as demonstrated in FIG. 10. In a similar manner, the click reactions described herein are applicable for direct library capture as shown in FIG. 11. Single stranded RNA can be directly captured onto the flow cell surface and sequencing adapters can be chemical ligated using click ligation, without the requirement to convert to the cDNA.

In a particular example of a modification of the process shown in Figure 11, a 3'-azido modified P7 sequence is prepared by reacting a P7 sequence (2 µM; P7 sequence: SEQ ID NO: 3, 5'-CAA GCA GAA GAC GGC ATA CGC-OH-3') with a 3'-azido-modified dGTP (prepared as in the preceding examples; 2 mM) in the presence of TdT (2 µM), TdT buffer, and CoCl₂ (0.25 mM) in water (20 µL) at 37 °C for 1 h, followed by reaction quench by heat inactivation of the enzyme at 75 °C for 10 min (yield, approx. 83%). Additionally, an oligonucleotide with a P5' sequence at the 3' end (SEQ ID NO: 4, 5'-TCGGTGGTCGCCGTATCATT-3') and a terminal alkyne-modified T nucleotide at the 5' end (prepared as described in the preceding examples; SEQ ID NO: 5, 5'-alkyne-T *GTA CAG TGA CGA GTG ACG ATA CAC ACG CGT CTC TGA CGC GCG CAT AGT ATC* GAT CTC GGT GGT CGC CGT ATC ATT-3' (italicized portion is oligonucleotide 1; underlined portion is the P5' sequence; bold portion comprises a triazole linkage between an 3'-azido A and a 5'-alkynyl T)) is hybridized to P5 sequences (SEQ ID NO: 6, 5' AAT GAT ACG GCG ACC ACC GA 3') on a solid support (such as a flow cell) comprising grafted P5 and modified P7 (SEQ ID NO: 7, 5' CAA GCA GAA GAC GGC ATA CGC 3') primers. The terminal alkyne units were coupled to the 3'-modified P7-dGTP under click chemistry conditions, including the 3'-azido modified P7 sequence (4 pmol, 2.0 µM (final reaction concentration, 1.8 µM), 0.002 equiv.), PMDETA (4.2 mmol), CuSO₄•5H₂O (0.16 M, 80 nmol, 400 equiv.), and sodium ascorbate (400 mg/mL, 2 M, 800 nmol, 4000 equiv.), at a total final volume of 21.8 µL, at -4 °C for 18 hours (yield, approx. 53%). A control construct of 3'-P5'-Oligonucleotide 1-P7-OH-3' with a standard phosphate linkage was also prepared (yield, approx. 47%). The control construct was also hybridized to a P5/P7 modified solid support.

Cluster generation reactions were performed on the two constructs, and yielded comparable imaging results at 200, 20, 3, and 1 pM concentrations, and after up to 56 amplification cycles on the flow cell surface. The chemically ligated construct also was successfully used as a template in a sequencing-by-synthesis (SBS) protocol, including amplification, linearization, blocking, deprotection, and polymerase-mediated base incorporation at the triazole site. Thus, the insertion of a modified triazole linkage does not hinder base incorporation and visualization of clusters in an SBS sequencing method.

Although the invention has been described with reference to the disclosed embodiments, those skilled in the art will readily appreciate that the specific examples and studies detailed above are only illustrative of the invention.

## Claims

1. A method of preparing a nucleic acid library from a sample comprising;
(i) reacting a sample comprising a plurality of cellular nucleic acids each having a terminal 3'-modified dideoxynucleotide (ddNTP) comprising a 3'-azido moiety, with a plurality of adaptor nucleic acids each comprising a terminal 5'-modified ddNTP comprising a 5'-alkynyl moiety, wherein the 3' functional moiety and 5' functional moiety react in a click chemistry reaction to form a modified backbone linkage comprising the structure 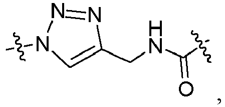 thereby forming a plurality of ligated-nucleic acids; and
(ii) amplifying the plurality of ligated-nucleic acids thereby preparing a nucleic acid library from the sample, wherein the terminal 3'-modified ddNTP is incorporated into each of the plurality of cellular nucleic acids by contacting the plurality of cellular nucleic acids with a template independent polymerase, and wherein the template independent polymerase is TdT.

2. The method of claim 1, wherein TdT incorporates the terminal 3'-modified ddNTP at a yield of at least 70%

3. The method of claim 2, wherein the terminal 3'-modified ddNTP is incorporated by the template independent polymerase in a reaction performed at a temperature of about 20 °C to about 40 °C.

4. The method of any one of claims 1-3, wherein the click chemistry reaction comprises copper catalyzed azide-alkyne cycloaddition (CuAAC).

5. The method of any one of claims 1-4, wherein the cellular nucleic acids comprise cellular nucleic acid fragments, optionally obtained from genomic DNA (gDNA), mitochondrial DNA, or plastomic DNA.

6. The method of any one of claims 1-5, wherein the cellular nucleic acids are attached to a solid support before the reacting, the adaptor nucleic acids are attached to a solid support before the reacting, the cellular nucleic acids are attached to a solid support before the terminal 3'-modified ddNTP is incorporated into the cellular nucleic acids, or the adaptor nucleic acids are attached to a solid support before the terminal 5'-modified ddNTP is incorporated into the adaptor nucleic acids.

7. The method of any one of claims 1-6, further comprising amplifying the plurality of cellular nucleic acids by polymerase chain reaction (PCR) before incorporating the terminal 3'-modified ddNTP.

8. The method of any one of claims 1-5, further comprising contacting the plurality of ligated-nucleic acids to a solid support under conditions for hybridization, wherein the solid support comprises (1) a plurality of capture primers each having a nucleic acid sequence complementary to the plurality of adaptor nucleic acids and (2) a plurality of 3'-universal primers optionally further comprising extending the plurality of capture primers to produce a plurality of immobilized ligated-nucleic acids optionally further comprising annealing the plurality of universal primers to the immobilized ligated-nucleic acids.

9. A method of capturing DNA obtained from a limited number of cells for DNA library preparation, the method comprising
(i) reacting a sample obtained from a limited number of cells, the sample comprising a plurality of cellular DNA fragments comprising a terminal 3'-modified dideoxynucleotide (ddNTP) comprising a 3'-azido moiety, wherein the terminal 3'-modified ddNTP is incorporated into each of the plurality of cellular DNA fragments by contacting the plurality of cellular DNA fragments with a template independent polymerase, with a plurality of adaptor nucleic acids each comprising a terminal 5'-modified ddNTP comprising a 5'-alkynyl moiety, wherein the 3'-azido moiety and 5'-alkynyl moiety react in a click chemistry reaction to form a plurality of ligated-nucleic acids comprising a modified backbone linkage comprising the structure 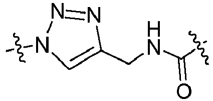 , and wherein the independent polymerase is TdT; and
(ii) contacting the plurality of ligated-nucleic acids to a solid support under conditions for hybridization, wherein the solid support comprises (1) a plurality of capture primers having a nucleic acid sequence complementary to the plurality of adaptor nucleic acids, thereby capturing DNA obtained from a single cell.

10. A method of selectively cleaving a single strand of a double stranded polynucleotide sequence, the method comprising:
(i) preparing a template strand by reacting a first polynucleotide comprising a terminal 3'-modified dideoxynucleotide (ddNTP) comprising a 3'-azido moiety with a second polynucleotide comprising a terminal 5'-modified ddNTP comprising a 5'-alkynyl moiety, wherein the terminal 3'-modified ddNTP is incorporated into the first polynucleotide by contacting the first polynucleotide with a template independent polymerase, wherein the 3'-azido moiety and 5'-alkynyl moiety react in a click chemistry reaction to form a modified backbone linkage comprising the structure 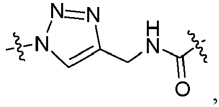 wherein the template strand comprises a first restriction site that comprises the modified backbone linkage and wherein the independent polymerase is TdT;
(ii) extending the first or second polynucleotide to produce a double stranded nucleic acid, wherein the complementary strand of the double stranded nucleic acid comprises a second restriction site complementary to the first restriction site;
(iii) contacting the double stranded nucleic acid with a nucleic acid-cleaving enzyme;
(iv) cleaving the double stranded nucleic acid with the nucleic acid-cleaving enzyme, wherein the nucleic acid-cleaving enzyme recognizes the first and second restriction sites and cleaves only at the second restriction site, forming a 5'-primer sequence and a 3'-strand.

11. The method of claim 10, wherein the first polynucleotide is part of a plurality of polynucleotides and/or wherein the double stranded nucleic acid is produced by extending from the second polynucleotide.

12. The method of claim 10 or 11, wherein the DNA cleaving enzyme is a restriction endonuclease (REase) or a nicking endonuclease (NEase).

## Patentansprüche

1. Ein Verfahren zum Herstellen einer Nukleinsäurebibliothek aus einer Probe, das Folgendes beinhaltet:
(i) Zur-Reaktion-Bringen einer Probe, beinhaltend eine Vielzahl von zellulären Nukleinsäuren, die jeweils ein terminales 3'-modifiziertes Didesoxynukleotid (ddNTP) aufweisen, das eine 3'-Azidoeinheit beinhaltet, mit einer Vielzahl von Adapternukleinsäuren, die jeweils ein terminales 5'-modifiziertes ddNTP beinhalten, das eine 5'-Alkinyleinheit beinhaltet, wobei die funktionelle 3'-Einheit und die funktionelle 5'-Einheit in einer Klick-Chemie-Reaktion miteinander reagieren, um eine modifizierte Rückgratverknüpfung zu bilden, die die folgende Struktur beinhaltet: 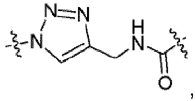 wodurch eine Vielzahl von ligierten Nukleinsäuren gebildet wird; und
(ii) Amplifizieren der Vielzahl von ligierten Nukleinsäuren, wodurch eine Nukleinsäurebibliothek aus der Probe hergestellt wird, wobei das terminale 3'-modifizierte ddNTP in jede der Vielzahl von zellulären Nukleinsäuren inkorporiert wird, indem die Vielzahl von zellulären Nukleinsäuren mit einer matrizenunabhängigen Polymerase in Kontakt gebracht wird, und wobei die matrizenunabhängige Polymerase TdT ist.

2. Verfahren gemäß Anspruch 1, wobei TdT das terminale 3'-modifizierte ddNTP in einer Menge von mindestens 70 % inkorporiert.

3. Verfahren gemäß Anspruch 2, wobei das terminale 3'-modifizierte ddNTP durch die matrizenunabhängige Polymerase in einer Reaktion inkorporiert wird, die bei einer Temperatur von etwa 20 °C bis etwa 40 °C durchgeführt wird.

4. Verfahren gemäß einem der Ansprüche 1-3, wobei die Klick-Chemie-Reaktion kupferkatalysierte Azid-Alkin-Cycloaddition (CuAAC) beinhaltet.

5. Verfahren gemäß einem der Ansprüche 1-4, wobei die zellulären Nukleinsäuren Fragmente zellulärer Nukleinsäure beinhalten, die optional aus genomischer DNA (gDNA), mitochondrialer DNA oder plastomischer DNA erhalten werden.

6. Verfahren gemäß einem der Ansprüche 1-5, wobei die zellulären Nukleinsäuren vor dem Zur-Reaktion-Bringen an einem festen Träger befestigt werden, die Adapternukleinsäuren vor dem Zur-Reaktion-Bringen an einem festen Träger befestigt werden, die zellulären Nukleinsäuren an einem festen Träger befestigt werden, bevor das terminale 3'-modifizierte ddNTP in die zellulären Nukleinsäuren inkorporiert wird, oder die Adapternukleinsäuren an einem festen Träger befestigt werden, bevor das terminale 5'-modifizierte ddNTP in die Adapternukleinsäuren inkorporiert wird.

7. Verfahren gemäß einem der Ansprüche 1-6, ferner beinhaltend das Amplifizieren der Vielzahl von zellulären Nukleinsäuren durch Polymerase-Kettenreaktion (PCR) vor dem Inkorporieren des terminalen 3'-modifizierten ddNTP.

8. Verfahren gemäß einem der Ansprüche 1-5, ferner beinhaltend das In-Kontakt-Bringen der Vielzahl von ligierten Nukleinsäuren mit einem festen Träger unter Hybridisierungsbedingungen, wobei der feste Träger (1) eine Vielzahl von Fangprimern, die jeweils eine Nukleinsäuresequenz aufweisen, die komplementär zu der Vielzahl von Adapternukleinsäuren ist, und (2) eine Vielzahl von 3'-universellen Primern beinhaltet, optional ferner beinhaltend das Verlängern der Vielzahl von Fangprimern, um eine Vielzahl von immobilisierten ligierten Nukleinsäuren zu erzeugen, optional ferner beinhaltend das Annealing der Vielzahl von universellen Primern an die immobilisierten ligierten Nukleinsäuren.

9. Ein Verfahren zum Einfangen von DNA, die aus einer begrenzten Anzahl von Zellen zur Herstellung einer DNA-Bibliothek erhalten wurde, wobei das Verfahren Folgendes beinhaltet:
(i) Zur-Reaktion-Bringen einer aus einer begrenzten Anzahl von Zellen erhaltenen Probe, wobei die Probe eine Vielzahl von Fragmenten zellulärer DNA beinhaltet, die ein terminales 3'-modifiziertes Didesoxynukleotid (ddNTP) beinhalten, das eine 3'-Azidoeinheit beinhaltet, wobei das terminale 3'-modifizierte ddNTP in jedes der Vielzahl von Fragmenten zellulärer DNA inkorporiert wird, indem die Vielzahl von Fragmenten zellulärer DNA mit einer matrizenunabhängigen Polymerase in Kontakt gebracht wird, wobei eine Vielzahl von Adapternukleinsäuren jeweils ein terminales 5'-modifiziertes ddNTP beinhaltet, das eine 5'-Alkinyleinheit beinhaltet, wobei die 3'-Azidoeinheit und die 5'-Alkinyleinheit in einer Click-Chemie-Reaktion miteinander reagieren, um eine Vielzahl von ligierten Nukleinsäuren zu bilden, die eine modifizierte Rückgratverknüpfung beinhalten, die die folgende Struktur beinhaltet: 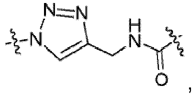 und wobei die unabhängige Polymerase TdT ist; und
(ii) In-Kontakt-Bringen der Vielzahl von ligierten Nukleinsäuren mit einem festen Träger unter Hybridisierungsbedingungen, wobei der feste Träger (1) eine Vielzahl von Fangprimern, die eine Nukleinsäuresequenz aufweisen, die komplementär zu der Vielzahl von Adapternukleinsäuren ist, beinhaltet, wodurch DNA, die aus einer einzigen Zelle erhalten wurde, eingefangen wird.

10. Ein Verfahren zum selektiven Spalten eines Einzelstrangs einer doppelsträngigen Polynukleotidsequenz, wobei das Verfahren Folgendes beinhaltet:
(i) Herstellen eines Matrizenstrangs durch Zur-Reaktion-Bringen eines ersten Polynukleotids, beinhaltend ein terminales 3'-modifiziertes Didesoxynukleotid (ddNTP), das eine 3'-Azidoeinheit beinhaltet, mit einem zweiten Polynukleotid, beinhaltend ein terminales 5'-modifiziertes ddNTP, beinhaltend eine 5'-Alkinyleinheit, wobei das terminale 3'-modifizierte ddNTP in das erste Polynukleotid inkorporiert wird, indem das erste Polynukleotid mit einer matrizenunabhängigen Polymerase in Kontakt gebracht wird, wobei die 3'-Azidoeinheit und die 5'-Alkinyleinheit in einer Klick-Chemie-Reaktion miteinander reagieren, um eine modifizierte Rückgratverknüpfung zu bilden, die die folgende Struktur beinhaltet: 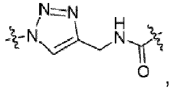 wobei der Matrizenstrang eine erste Restriktionsstelle beinhaltet, die die modifizierte Rückgratverknüpfung beinhaltet, und wobei die unabhängige Polymerase TdT ist;
(ii) Verlängern des ersten oder des zweiten Polynukleotids, um eine doppelsträngige Nukleinsäure zu erzeugen, wobei der komplementäre Strang der doppelsträngigen Nukleinsäure eine zweite Restriktionsstelle beinhaltet, die komplementär zu der ersten Restriktionsstelle ist;
(iii) In-Kontakt-Bringen der doppelsträngigen Nukleinsäure mit einem nukleinsäurespaltenden Enzym;
(iv) Spalten der doppelsträngigen Nukleinsäure mit dem nukleinsäurespaltenden Enzym, wobei das nukleinsäurespaltende Enzym die erste und die zweite Restriktionsstelle erkennt und nur an der zweiten Restriktionsstelle spaltet, wodurch eine 5'-Primersequenz und ein 3'-Strang gebildet werden.

11. Verfahren gemäß Anspruch 10, wobei das erste Polynukleotid Teil einer Vielzahl von Polynukleotiden ist und/oder wobei die doppelsträngige Nukleinsäure durch Verlängern aus dem zweiten Polynukleotid erzeugt wird.

12. Verfahren gemäß Anspruch 10 oder 11, wobei das DNA-spaltende Enzym eine Restriktionsendonuklease (REase) oder eine Nicking-Endonuklease (NEase) ist.

## Revendications

1. Un procédé de préparation d'une banque d'acides nucléiques à partir d'un échantillon comprenant :
(i) la mise en réaction d'un échantillon comprenant une pluralité d'acides nucléiques cellulaires ayant chacun un didésoxynucléotide (ddNTP) modifié en 3' terminal comprenant une partie azido en 3', avec une pluralité d'acides nucléiques adaptateurs comprenant chacun un ddNTP modifié en 5' terminal comprenant une partie alcynyle en 5', où la partie fonctionnelle en 3' et la partie fonctionnelle en 5' réagissent dans une réaction de chimie clic afin de former une liaison de squelette modifié comprenant la structure 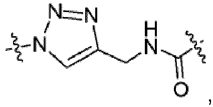 formant par là une pluralité d'acides nucléiques ligaturés ; et
(ii) l'amplification de la pluralité d'acides nucléiques ligaturés, préparant par là une banque d'acides nucléiques à partir de l'échantillon, où le ddNTP modifié en 3' terminal est incorporé au sein de chaque acide de la pluralité d'acides nucléiques cellulaires par la mise en contact de la pluralité d'acides nucléiques cellulaires avec une polymérase indépendante d'une matrice, et où la polymérase indépendante d'une matrice est la TdT.

2. Le procédé de la revendication 1, où la TdT incorpore le ddNTP modifié en 3' terminal à raison d'un rendement d'au moins 70 %.

3. Le procédé de la revendication 2, où le ddNTP modifié en 3' terminal est incorporé par la polymérase indépendante d'une matrice dans une réaction mise en œuvre à une température allant d'environ 20 °C à environ 40 °C.

4. Le procédé de l'une quelconque des revendications 1 à 3, où la réaction de chimie clic comprend une cycloaddition azoture/alcyne catalysée par le cuivre (CuAAC).

5. Le procédé de l'une quelconque des revendications 1 à 4, où les acides nucléiques cellulaires comprennent des fragments d'acides nucléiques cellulaires, obtenus facultativement à partir d'ADN génomique (ADNg), d'ADN mitochondrial, ou d'ADN plastomique.

6. Le procédé de l'une quelconque des revendications 1 à 5, où les acides nucléiques cellulaires sont attachés à un support solide avant la mise en réaction, les acides nucléiques adaptateurs sont attachés à un support solide avant la mise en réaction, les acides nucléiques cellulaires sont attachés à un support solide avant que le ddNTP modifié en 3' terminal soit incorporé au sein des acides nucléiques cellulaires, ou les acides nucléiques adaptateurs sont attachés à un support solide avant que le ddNTP modifié en 5' terminal soit incorporé au sein des acides nucléiques adaptateurs.

7. Le procédé de l'une quelconque des revendications 1 à 6, comprenant en sus l'amplification de la pluralité d'acides nucléiques cellulaires par une réaction en chaîne par polymérase (PCR) avant l'incorporation du ddNTP modifié en 3' terminal.

8. Le procédé de l'une quelconque des revendications 1 à 5, comprenant en sus la mise en contact de la pluralité d'acides nucléiques ligaturés avec un support solide dans des conditions propices à une hybridation, où le support solide comprend (1) une pluralité d'amorces de capture ayant chacune une séquence d'acide nucléique complémentaire à la pluralité d'acides nucléiques adaptateurs et (2) une pluralité d'amorces universelles en 3' comprenant facultativement en sus l'extension de la pluralité d'amorces de capture afin de produire une pluralité d'acides nucléiques ligaturés immobilisés comprenant facultativement en sus l'annelage de la pluralité d'amorces universelles aux acides nucléiques ligaturés immobilisés.

9. Un procédé de capture d'ADN obtenu à partir d'un nombre limité de cellules pour la préparation d'une banque d'ADN, le procédé comprenant
(i) la mise en réaction d'un échantillon obtenu à partir d'un nombre limité de cellules, l'échantillon comprenant une pluralité de fragments d'ADN cellulaire comprenant un didésoxynucléotide (ddNTP) modifié en 3' terminal comprenant une partie azido en 3', où le ddNTP modifié en 3' terminal est incorporé au sein de chaque fragment de la pluralité de fragments d'ADN cellulaire par mise en contact de la pluralité de fragments d'ADN cellulaire avec une polymérase indépendante d'une matrice, avec une pluralité d'acides nucléiques adaptateurs comprenant chacun un ddNTP modifié en 5' terminal comprenant une partie alcynyle en 5', où la partie azido en 3' et la partie alcynyle en 5' réagissent dans une réaction de chimie clic afin de former une pluralité d'acides nucléiques ligaturés comprenant une liaison de squelette modifié comprenant la structure 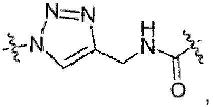 et où la polymérase indépendante est la TdT ; et
(ii) la mise en contact de la pluralité d'acides nucléiques ligaturés avec un support solide dans des conditions propices à une hybridation, où le support solide comprend (1) une pluralité d'amorces de capture ayant une séquence d'acide nucléique complémentaire à la pluralité d'acides nucléiques adaptateurs, capturant par là l'ADN obtenu à partir d'une seule cellule.

10. Un procédé de clivage sélectif d'un simple brin d'une séquence polynucléotidique double brin, le procédé comprenant :
(i) la préparation d'un brin matrice par la mise en réaction d'un premier polynucléotide comprenant un didésoxynucléotide (ddNTP) modifié en 3' terminal comprenant une partie azido en 3' avec un deuxième polynucléotide comprenant un ddNTP modifié en 5' terminal comprenant une partie alcynyle en 5', où le ddNTP modifié en 3' terminal est incorporé au sein du premier polynucléotide par mise en contact du premier polynucléotide avec une polymérase indépendante d'une matrice, où la partie azido en 3' et la partie alcynyle en 5' réagissent dans une réaction de chimie clic afin de former une liaison de squelette modifié comprenant la structure 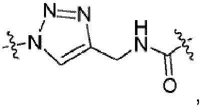 où le brin matrice comprend un premier site de restriction qui comprend la liaison de squelette modifié et où la polymérase indépendante est la TdT ;
(ii) l'extension du premier ou deuxième polynucléotide afin de produire un acide nucléique double brin, où le brin complémentaire de l'acide nucléique double brin comprend un deuxième site de restriction complémentaire au premier site de restriction ;
(iii) la mise en contact de l'acide nucléique double brin avec une enzyme de clivage d'acide nucléique ;
(iv) le clivage de l'acide nucléique double brin avec l'enzyme de clivage d'acide nucléique, où l'enzyme de clivage d'acide nucléique reconnaît les premier et deuxième sites de restriction et clive uniquement au niveau du deuxième site de restriction, formant une séquence d'amorce en 5' et un brin en 3'.

11. Le procédé de la revendication 10, où le premier polynucléotide fait partie d'une pluralité de polynucléotides et/ou où l'acide nucléique double brin est produit par extension à partir du deuxième polynucléotide.

12. Le procédé de la revendication 10 ou de la revendication 11, où l'enzyme de clivage d'ADN est une endonucléase de restriction (REase) ou une entaillase *(nicking endonuclease,* NEase).
